# EUROPEAN PATENT APPLICATION

(11) **EP 4 631 496 A1**
(43) Date of publication of application: **15.10.2025**
(21) Application number: 23900012.8
(22) Date of filing: 06.12.2023
(51) Int. Cl.: A61K 9/00, A61K 31/454, A61P 35/00

(54) **SOLID DISPERSION, PREPARATION METHOD THEREFOR, AND USE THEREOF**

(30) Priority: 07.12.2022 CN 202211567771
(71) Applicant: Hangzhou Glubio Pharmaceutical Co., Ltd., Hangzhou, Zhejiang 310018 (CN)
(72) Inventor: FU, Liqiang, Hangzhou, Zhejiang 310018 (CN); QI, Zude, Hangzhou, Zhejiang 310018 (CN); WANG, Zheng, Hangzhou, Zhejiang 310018 (CN); KONG, Fandi, Hangzhou, Zhejiang 310018 (CN)
(74) Representative: Ipsilon
(86) International application number: PCT/CN2023/136781
(87) International publication number: WO 2024/120438

(57) **Abstract**

A solid dispersion, a preparation method therefor, and use thereof. The solid dispersion comprises an active ingredient: the compound of formula A or a derivative thereof, a crystal form thereof, an amorphous form thereof, or a pharmaceutically acceptable salt thereof, a hydrate thereof, or a solvate thereof, and comprises a carrier. The solid dispersion can significantly improve the oral bioavailability of the active ingredient and has good solid stability, causing the active ingredient to show higher plasma exposure in a rat.

## Description

### TECHNICAL FIELD

The present invention belongs to the field of medicine, and particularly relates to a solid dispersion, a preparation method therefor and use thereof.

### BACKGROUND

Casein kinase 1a (CK1α), encoded by the gene CSNK1A1, is a ubiquitously expressed serine/threonine protein kinase in the CK1 kinase family. CK1α participates in regulating a variety of physiological and pathological processes in cells and coordinates the orderly progress of life through different signal transduction pathways (Jiang et al., Cell Commun Signal (2018) 16: 23). For example, CK1α, as a key regulator of the Wnt/β-catenin pathway, directly phosphorylates β-catenin at Ser45, making it a target for protein degradation (Liu et al., Cell (2002) 108: 837-847). CK1α is also considered to regulate the protein stability of the tumor suppressor p53 by regulating the activity of the MDM2/MDMX E3 ligase active complex (Huart et al., J Biol Chem (2009) 284: 32384-94; Wu et al., Mol Cell Biol (2012) 32:4821-4832). It is reported that CK1α is overexpressed in many types of human cancers. However, the exact role of CK1α in the development of each tumor type has not been clearly elucidated (Richter et al., BMC Cancer (2018) 18: 140). The Cancer Dependency Map (DepMap) database showed that inactivation of CK1α by CRISPR/cas9-mediated gene knockout or shRNA-mediated gene inhibition significantly reduced the proliferation and/or survival of many cancer cell lines in multiple cancer types (Tshemiak et al., Cell (2017) 170:564-576; Behan et al., Nature (2019) 568: 511-516). In addition, inhibition of CK1α activity by using shRNA interference or D4476 (a CK1α inhibitor) may effectively inhibit the progression of MLL-AF9 leukemia and had little effect on normal hematopoietic stem and progenitor cells (HSPCs) (Jaras et al, J Exp Med (2014) 211(4): 605-612). In summary, these data indicate that CK1α is a potential target for treating indications of hematological malignancies and solid tumor.

The compound of formula A is a new generation of CK1α selective molecular glue degrader and is currently in the early stage of clinical research.

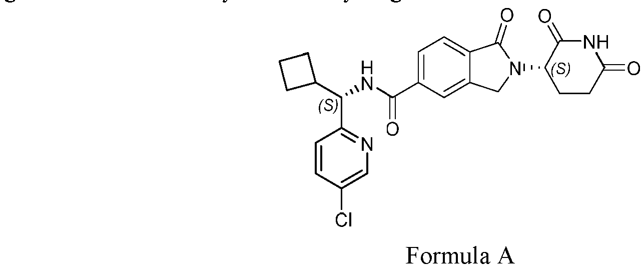

However, there is no research or report on the polymorphism, amorphous form, formulation, etc. of the compound of formula A.

Therefore, in the process of new drug development, it is necessary to screen the drug compounds comprehensively and consider multiple factors. In particular, for the above-mentioned compound of formula A for treating proliferative disorders, developing a dosage form of the compound or a derivative, crystalline form, amorphous form, or pharmaceutically acceptable salt, hydrate or solvate thereof that may have pharmaceutical value has potential medicinal and clinical value for improving the stability, solubility, bioavailability and other properties of the compound.

### SUMMARY OF THE INVENTION

The present invention provides a solid dispersion with the compound of formula A or a derivative, a crystalline form, an amorphous form, or a pharmaceutically acceptable salt, a hydrate or a solvate thereof as an active ingredient. The solid dispersion allows the active ingredient, the compound of formula A, have great oral bioavailability, which is of great value to the development and production of new drugs.

In the following description, certain specific details are set forth in order to provide a thorough understanding of various embodiments of the present invention. However, it will be understood by those skilled in the art that the present invention may be practiced without these details. The following description of certain embodiments are carried out with the understanding that is considered as an example of the claimed subject matter of the present invention, and is not intended to limit the appended claims to the displayed specific embodiments. The titles used throughout the present invention are provided only for convenience and should not be interpreted as limiting the claims in any way. The embodiment shown under any title may be combined with an embodiment displayed under any other title.

**In the first aspect,** the present invention provides a solid dispersion comprising an active ingredient and a carrier, wherein the active ingredient is one or more of the compound of formula A or a derivative, a crystalline form, an amorphous form, or a pharmaceutically acceptable salt, a hydrate or a solvate thereof, and the carrier is selected from one or more of: homopolymer and copolymer of N-vinyl lactam, cellulose derivative, graft copolymer, polyalkylene oxide with a high molecular weight, polyacrylate and polymethacrylate, polyacrylamide, polyvinyl acetate, oligosaccharide or polysaccharide or a copolymer thereof, andacrylic acid copolymer.

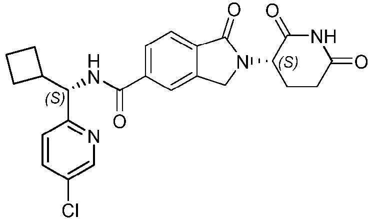

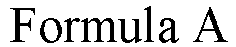

In some preferred embodiments, the homopolymer and copolymer of N-vinyl lactam is povidone, or a copolymer of PVP and polyvinyl acetate; preferably, the povidone is PVP K30; preferably, the copolymer of PVP and polyvinyl acetate is PVP VA64.

In some preferred embodiments, the cellulose derivative is hydroxypropyl methylcellulose acetate succinate, hydroxypropyl methylcellulose, hydroxypropyl cellulose, methyl cellulose, or ethyl cellulose; preferably, the hydroxypropyl methylcellulose acetate succinate is one or more of HPMCAS 716G, HPMCAS 912G, HPMCAS 126G, HPMCAS 716F, HPMCAS 912F, HPMCAS 126F, HPMCAS LG, HPMCAS MG, HPMCAS HG, HPMCAS LF, HPMCAS MF and HPMCAS HF; preferably, the hydroxypropyl methylcellulose is HPMC E3; preferably, the hydroxypropyl cellulose is HPC SSL.

In some preferred embodiments, the polyalkylene oxide with a high molecular weight is polyethylene oxide, polypropylene oxide, and copolymer of ethylene oxide and propylene oxide (poloxamer).

In some preferred embodiments, the polyacrylate and polymethacrylate are methacrylic acid / ethyl acrylate copolymer, methacrylic acid / methyl methacrylate copolymer, butyl methacrylate / dimethylaminoethyl 2-methacrylate copolymer, poly(hydroxyalkyl acrylate) and poly(hydroxyalkyl methacrylate).

In some preferred embodiments, the polyvinyl acetate is a copolymer of vinyl acetate and crotonic acid, partially hydrolyzed polyvinyl acetate, and polyvinyl alcohol.

In some preferred embodiments, the oligosaccharide or polysaccharide is carrageenan, galactomannan and xanthan gum, and a mixture of two or more thereof.

In some preferred embodiments, the carrier of the solid dispersion comprises one or more following polymer carriers selected from: polyvinyl pyrrolidone, hydroxypropyl methylcellulose, and mixtures thereof. A specific embodiment of a useful copovidone consists of about 60% N-vinyl pyrrolidone and about 40% vinyl acetate monomers. A specific embodiment of a useful povidone is a povidone having a K value (a viscosity measurement standard for aqueous povidone solution) of about 30.

In some preferred embodiments, the acrylic copolymer is a methacrylic acid copolymer and a methacrylate copolymer; preferably, the methacrylic acid copolymer and the methacrylate copolymer is Eudragit; preferably, the Eudragit is one or more selected from Eudragit L100, Eudragit E100, Eudragit S100, Eudragit L100-55, and Eudragit EPO.

In some preferred embodiments, the graft copolymer is Soluplus.

In some preferred embodiments, the pharmaceutically acceptable salt of the compound of formula A is hydrochloride, hydrobromide, hydroiodide, sulfate, bisulfate, 2-hydroxyethanesulfonate, phosphate, hydrogenphosphate, acetate, adipate, alginate, lysine, arginine, histidine, aspartate, benzoate, butyrate, camphorate, camphorsulfonate, digluconate, glycerophosphate, hemisulfate, heptanoate, hexanoate, formate, succinate, fumarate, maleate, ascorbate, isethionate, salicylate, methanesulfonate, mesitylenesulfonate, naphthalenesulfonate, nicotinate, 2-naphthalenesulfonate, oxalate, pamoate, pectinate, persulfate, 3-phenylpropionate, picrate, pivalate, propionate, trichloroacetate, trifluoroacetate, glutamate, bicarbonate, undecanoate, lactate, citrate, tartrate, gluconate, edisylate, benzenesulfonate, L-tartrate, maleate, sodium salt, potassium salt, choline salt, tromethamine salt, calcium salt or p-toluenesulfonate, preferably phosphate, sulfate, L-tartrate, hydrochloride, maleate, hydrobromide, methanesulfonate, lysine salt, arginine salt, histidine salt, sodium salt, potassium salt, choline salt, tromethamine salt, or calcium salt.

In some preferred embodiments, the hydrate of the compound of formula A is hemihydrate, monohydrate, dihydrate, trihydrate, tetrahydrate, pentahydrate, hexahydrate, heptahydrate, octahydrate, nonahydrate, decahydrate, undecahydrate or dodecahydrate.

In some preferred embodiments, the carrier is selected from one or more of polyvidone, copolymer of PVP and polyvinyl acetate, hydroxypropyl methylcellulose acetate succinate, hydroxypropyl methylcellulose, hydroxypropyl cellulose, acrylic acid copolymer and graft copolymer.

In some preferred embodiments, the carrier is selected from one or more of PVP K30, PVP VA64, Soluplus, HPMC E3, HPMCAS MG, HPMCAS HG, HPC SSL, and Eudragit L100.

In some preferred embodiments, the carrier is selected from one or more of PVP VA64, Soluplus and HPC SSL.

In some preferred embodiments, the carrier is HPC SSL.

The solid dispersion of the present invention is in an amorphous form. More importantly, the amorphous solid dispersion has great solid stability, can be stored for a long time, and may not potentially affect the performance of the drug product.

The amount of the carrier used in the present invention may be conventional in the art, and the weight ratio of the active ingredient to the carrier is 0.1:10 ~ 10:0.1.

In some preferred embodiments, the weight ratio of the active ingredient to the carrier is 1:10 ~ 10:1, preferably 1:4 ~ 1:1.

In some preferred embodiments, the weight ratio of the active ingredient to the carrier is 1:4, 3:7, 4:6 or 1:1.

In some preferred embodiments, the carrier is PVP VA64; preferably, the weight ratio of the active ingredient to PVP VA64 is 1:4.

In some preferred embodiments, the carrier is Soluplus; preferably, the weight ratio of the active ingredient to Soluplus is 1:4.

In some preferred embodiments, the carrier is HPC SSL; preferably, the weight ratio of the active ingredient to HPC SSL is 1:4, 3:7, 4:6 or 1:1.

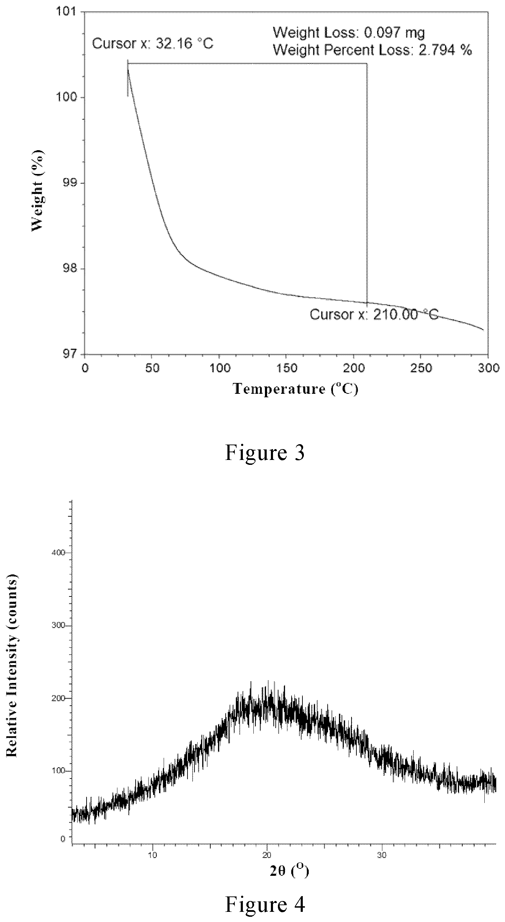

In some preferred embodiments, the active ingredient is an amorphous form of the compound of formula A having an XRPD pattern substantially as shown in Figure 4.

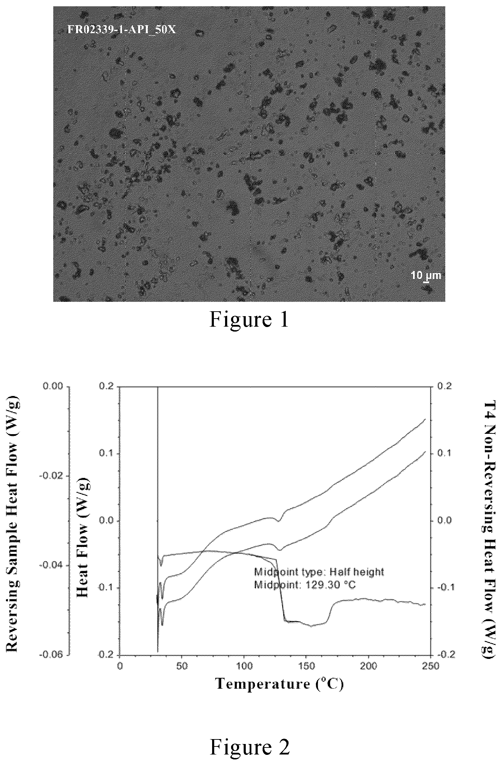

In another preferred embodiment, the amorphous form of the compound of formula A further optionally has one or more of the following features:
1) having a weight loss of 2.79 wt% before the temperature reaches 210 ± 2.0 °C in TGA pattern;
2) having a glass transition temperature at 129.30 ± 2.0 °C in mDSC pattern;
3) having a TGA pattern substantially as shown in Figure 3;
4) having a mDSC pattern essentially as shown in Figure 2.

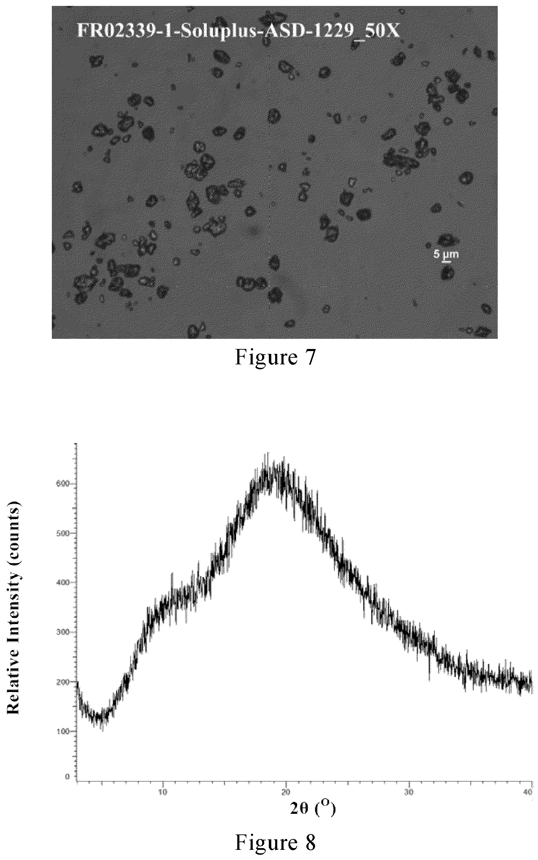

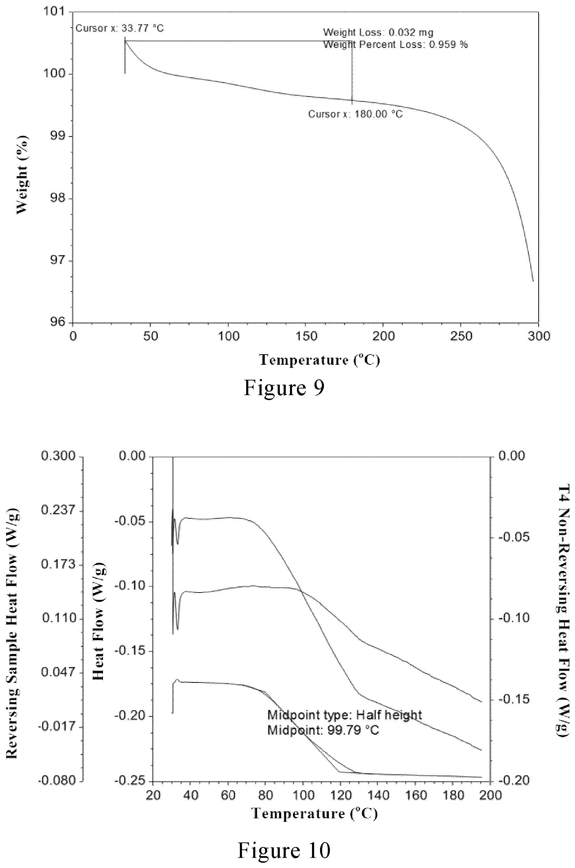

In some preferred embodiments, the active ingredient is the compound of formula A, and the solid dispersion of the compound of formula A and Soluplus in a weight ratio of 1:4 is an amorphous form as shown in PLM Figure 7, has an XRPD pattern substantially as shown in Figure 8, and optionally has one or more of the following features:
1) having a weight loss of 0.96 wt% before the temperature reaches 180.00 ± 2.0 °C in TGA pattern;
2) having a glass transition temperature at the middle temperature of 99.79 ± 2.0 °C in mDSC pattern;
3) having a TGA pattern substantially as shown in Figure 9; and/or
4) having a mDSC pattern substantially as shown in Figure 10.

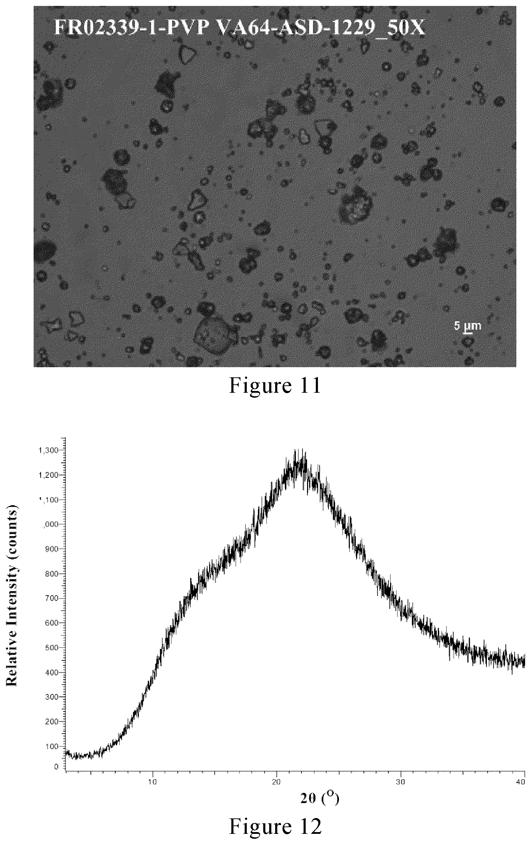

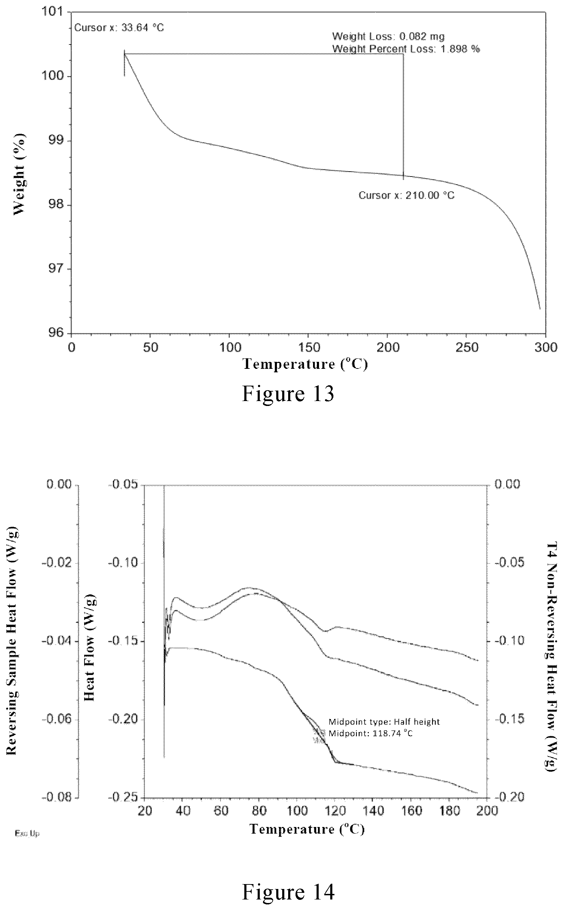

In some preferred embodiments, the active ingredient is the compound of formula A, and the solid dispersion of the compound of formula A and PVP VA64 in a weight ratio of 1:4 is an amorphous form as shown in PLM Figure 11, has an XRPD pattern substantially as shown in Figure 12, and optionally has one or more of the following features:
1) having a weight loss of 1.90 wt% before the temperature reaches 210 ± 2.0 °C in TGA pattern;
2) having a glass transition temperature at the middle temperature of 118.74 ± 2.0 °C in mDSC pattern;
3) having a TGA pattern substantially as shown in Figure 13; and/or
4) having a mDSC pattern substantially as shown in Figure 14.

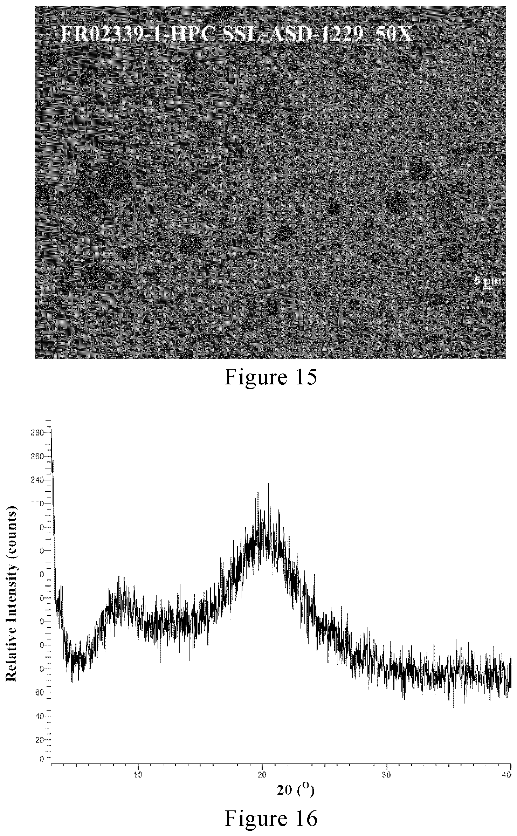

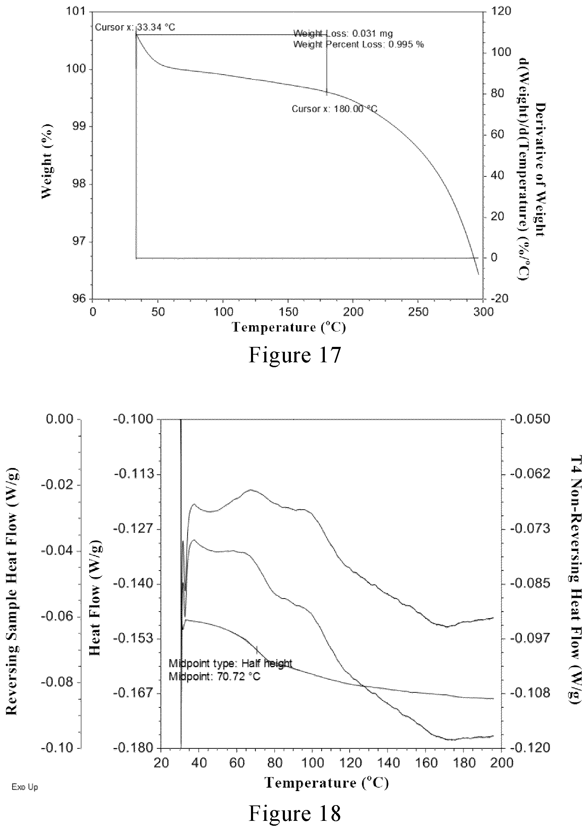

In some preferred embodiments, the active ingredient is the compound of formula A, and the solid dispersion of the compound of formula A and HPC SSL in a weight ratio of 1:4 is an amorphous form as shown in PLM Figure 15, has an XRPD pattern substantially as shown in Figure 16, and optionally has one or more of the following features:
1) having a 1.00 wt% weight loss before the temperature reaches 180 ± 2.0 °C in TGA pattern;
2) having a glass transition temperature at 70.72 ± 2.0 °C in mDSC pattern;
3) having a TGA pattern substantially as shown in Figure 17; and/or
4) having a mDSC pattern substantially as shown in Figure 18.

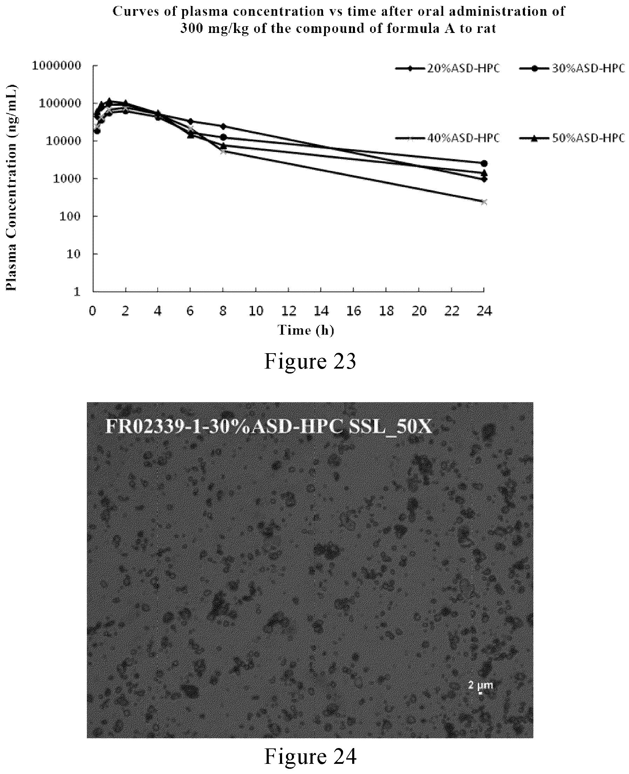

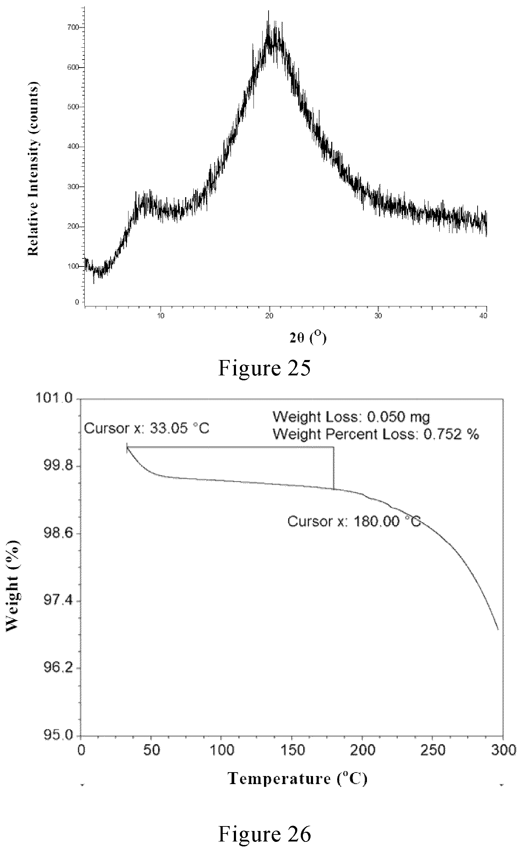

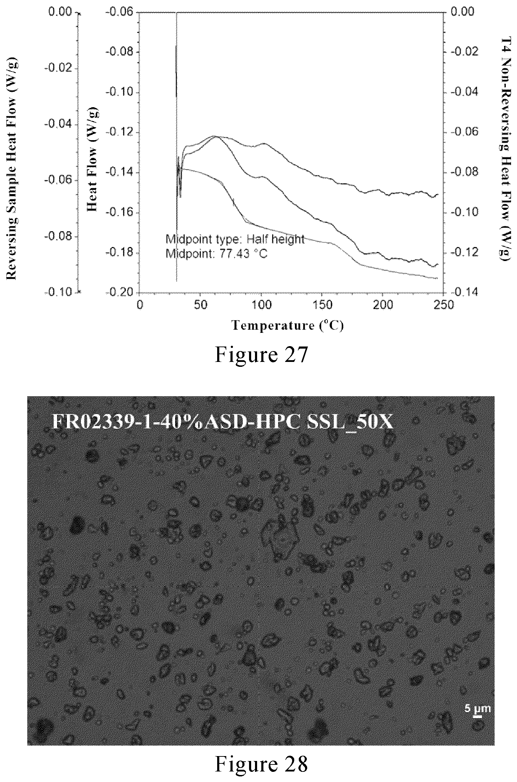

In some preferred embodiments, the active ingredient is the compound of formula A, and the solid dispersion of the compound of formula A and HPC SSL in a weight ratio of 3:7 is an amorphous form as shown in PLM Figure 24, has an XRPD pattern substantially as shown in Figure 25, and optionally has one or more of the following features:
1) having a weight loss of 0.75 wt% before the temperature reaches 180 ± 2.0 °C in TGA pattern;
2) having a glass transition temperature at 77.43 ± 2.0 °C in mDSC pattern;
3) having a TGA pattern substantially as shown in Figure 26; and/or
4) having a mDSC pattern substantially as shown in Figure 27.

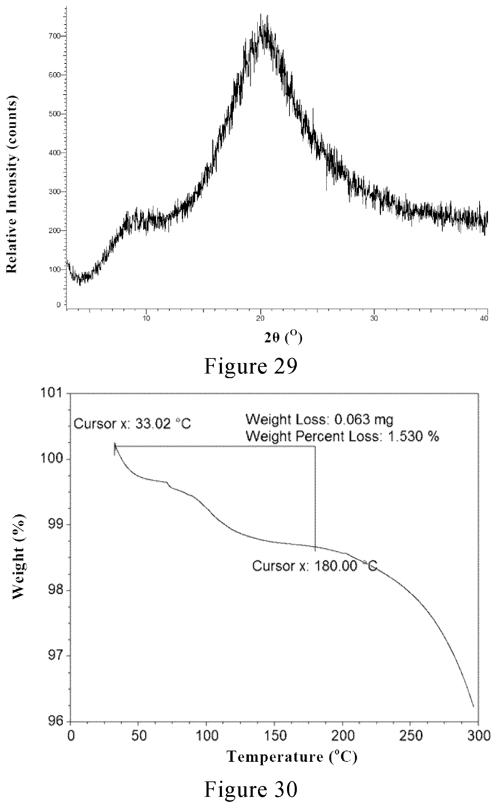

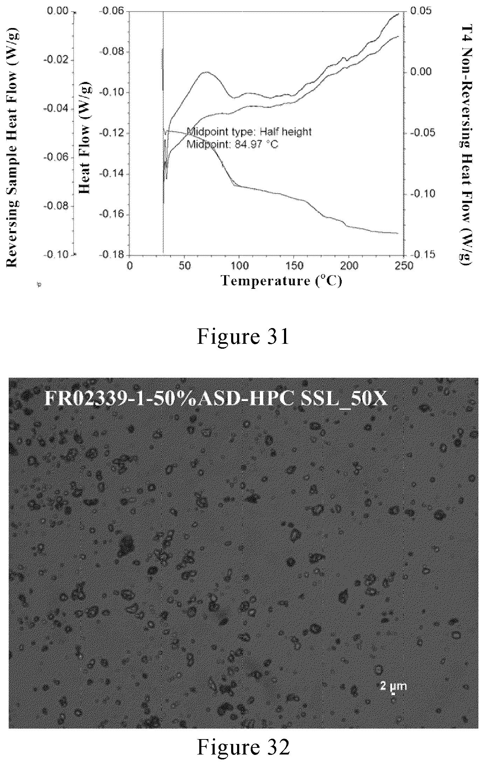

In some preferred embodiments, the active ingredient is the compound of formula A, and the solid dispersion of the compound of formula A and HPC SSL in a weight ratio of 4:6 is an amorphous form as shown in PLM Figure 28, has an XRPD pattern substantially as shown in Figure 29, and optionally has one or more of the following features:
1) having a weight loss of 1.53 wt% before the temperature reaches 180 ± 2.0 °C in TGA pattern;
2) having a glass transition temperature at 84.97 ± 2.0 °C in mDSC pattern;
3) having a TGA pattern substantially as shown in Figure 30; and/or
4) having a mDSC pattern substantially as shown in Figure 31.

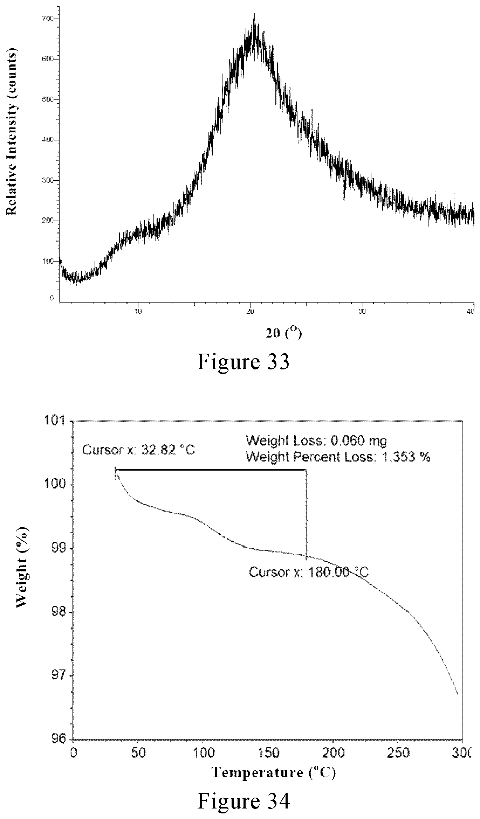

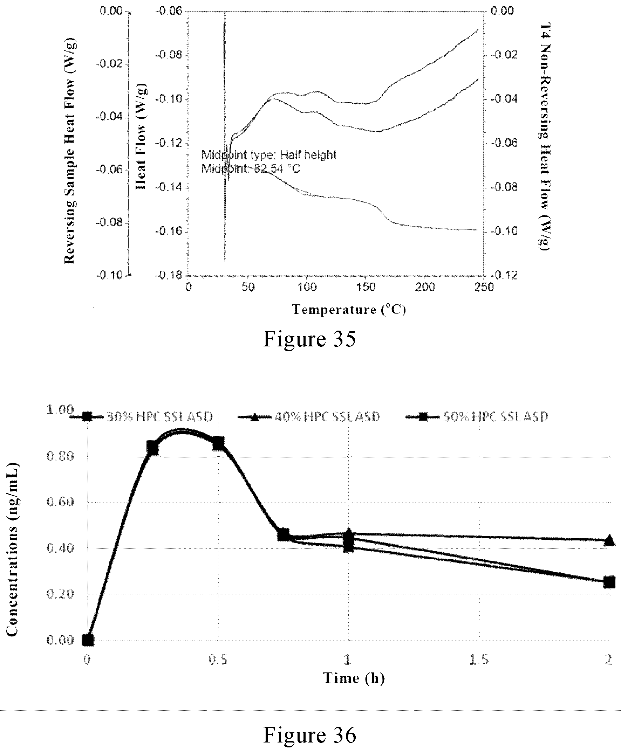

In some preferred embodiments, the active ingredient is the compound of formula A, and the solid dispersion of the compound of formula A and HPC SSL in a weight ratio of 1:1 is an amorphous form as shown in PLM Figure 32, has an XRPD pattern substantially as shown in Figure 33, and optionally has one or more of the following features:
1) having a weight loss of 1.35 wt% before the temperature reaches 180 ± 2.0 °C in TGA pattern;
2) having a glass transition temperature at 82.54 ± 2.0 °C in mDSC pattern;
3) having a TGA pattern substantially as shown in Figure 34; and/or
4) having a mDSC pattern substantially as shown in Figure 35.

In one embodiment, the solid dispersion has the following features:
the area under the curve of the active ingredient in the solid dispersion is 1.5 to 7 times, preferably 5 to 6 times, the area under the curve of the active ingredient when administered alone.

**In the second aspect,** the present invention provides a method for preparing the solid dispersion according to the first aspect, comprising steps of:
method 1
mixing one or more of the compound of formula A or a derivative, a crystalline form, an amorphous form thereof, or a pharmaceutically acceptable salt, a hydrate, or a solvate thereof, optionally with other components, with a solvent to form a solution or suspension, and removing the solvent to obtain the solid dispersion; or
method 2
mixing one or more of the compound of formula A or a derivative, a crystalline form, an amorphous form thereof, or a pharmaceutically acceptable salt, a hydrate, or a solvate thereof, optionally with other components, heating and then extruding to obtain the solid dispersion; or
method 3
mixing one or more of the compound of formula A or a derivative, a crystalline form, an amorphous form thereof, or a pharmaceutically acceptable salt, a hydrate, or a solvate thereof, optionally with other components, with a solvent and spray-drying to obtain the solid dispersion.

In some preferred embodiments, in method 1 or 3, the solvent is one or more selected from water, alcohol solvent, ester solvent, ketone solvent, halogenated hydrocarbon solvent, nitrile solvent and ether solvent, wherein the alcohol solvent is preferably ethanol and/or methanol; the ester solvent is preferably ethyl acetate; the ketone solvent is preferably acetone; the halogenated hydrocarbon solvent is preferably dichloromethane; the nitrile solvent is preferably acetonitrile; the ether solvent is preferably tetrahydrofuran; preferably, the solvent is dichloromethane and/or ethanol. Preferably, the solvent is acetone and/or water.

In some preferred embodiments, the weight to volume ratio of the "one or more of the compound of formula A or a derivative, a crystalline form, an amorphous form, or a pharmaceutically acceptable salt, a hydrate or a solvate thereof" to the solvent is (0.1~30):1 mg/mL, preferably (1~10):1 mg/mL, and more preferably 5:1.5 mg/mL.

In some preferred embodiments, the solvent is an alcohol solvent and/or a halogenated hydrocarbon solvent, wherein the alcohol solvent is preferably methanol; the halogenated hydrocarbon solvent is preferably dichloromethane; preferably, the solvent is dichloromethane and methanol, wherein the volume ratio of dichloromethane to methanol is preferably 9:1 ~ 1:1.

In some preferred embodiments, the solvent is an alcohol solvent and/or a halogenated hydrocarbon solvent, wherein the alcohol solvent is preferably ethanol; the halogenated hydrocarbon solvent is preferably dichloromethane; preferably, the solvent is dichloromethane and ethanol, wherein the volume ratio of dichloromethane to methanol is preferably 9:1 ~ 1: 1.

In some preferred embodiments, the solvent is acetone and water, wherein the volume ratio of acetone to water is preferably 7:1 ~ 10:1.

In some preferred embodiments, the inlet temperature of spray-drying air is set to 40 °C ~ 200 °C, preferably 80 °C ~ 120 °C.

**In the third aspect,** the present invention provides a pharmaceutical composition comprising the solid dispersion. the solid dispersion comprises an active ingredient and a carrier, wherein the active ingredient is one or more selected from the compound of formula A or a derivative, a crystalline form, an amorphous form, or a pharmaceutically acceptable salt, a hydrate or a solvate thereof, and the carrier is selected from one or more of a homopolymer and copolymer of N-vinyl lactam, a cellulose derivative, and/or a graft copolymer or an acrylic acid copolymer. The pharmaceutical composition comprises:
(1) a solid dispersion as described above; and (2) a pharmaceutically acceptable excipient.

In some preferred embodiments, the pharmaceutical composition may include other excipients, such as excipients used as fillers, binders, disintegrants, glidants and lubricants. Therefore, the solid pharmaceutical composition comprising the compound of formula A or a pharmaceutically acceptable salt thereof may further optionally comprise one or more conventional pharmaceutically acceptable excipients.

**In the fourth aspect,** the present invention provides a pharmaceutical formulation comprising the above-mentioned pharmaceutical composition; wherein the pharmaceutical formulation may be a solid formulation, or may be powder, granule, tablet, capsule, pill or film.

**In the fifth aspect,** the present invention provides a use of the above-mentioned solid dispersion, pharmaceutical composition or pharmaceutical formulation in the manufacture of a medicament for treating a proliferative disease. Preferably, the proliferative disease comprises breast cancer, colon cancer, brain cancer, prostate cancer, kidney cancer, pancreatic cancer, ovarian cancer, head and neck cancer, melanoma, colorectal cancer, gastric cancer, squamous cell carcinoma, small cell lung cancer, non-small cell lung cancer, testicular cancer, Merkel cell carcinoma, glioblastoma, neuroblastoma, lymphoid organ cancer and hematological malignancy including leukemia (acute lymphoblastic leukemia (ALL), acute myeloid leukemia (AML), chronic lymphocytic leukemia (CLL), chronic myeloid leukemia (CML), acute monocytic leukemia (AMOL), hairy cell leukemia (HCL), T cell prolymphocytic leukemia (T-PLL), large granular lymphocytic leukemia, adult T cell leukemia), lymphoma (small lymphocytic lymphoma (SLL), Hodgkin's lymphoma (nodular sclerosis, mixed cellular, lymphocyte-rich, lymphocyte depleted or not depleted, and nodular lymphocyte-predominant Hodgkin lymphoma), non-Hodgkin's lymphoma (all subtypes), chronic lymphocytic leukemia/small lymphocytic lymphoma, B cell prolymphocytic leukemia, lymphoplasmacytic lymphoma (such as Waldenström macroglobulinemia), splenic marginal zone lymphoma, plasma cell neoplasms (plasma cell myeloma, plasmacytoma, monoclonal immunoglobulin deposition disease, heavy chain disease), extranodal marginal zone B cell lymphoma (MALT lymphoma), lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, Burkitt lymphoma/leukemia, T cell prolymphocytic leukemia, T cell large granular lymphocytic leukemia, aggressive NK cell leukemia, adult T cell leukemia/lymphoma, extranodal NK/T cell lymphoma (nasal type), enteropathy-type T cell lymphoma, hepatosplenic T cell lymphoma, blastic NK cell lymphoma, mycosis fungoides/Sezary syndrome, primary cutaneous CD30-positive T cell lymphoma disease, primary cutaneous anaplastic large cell lymphoma, lymphomatoid papulosis, angioimmunoblastic T cell lymphoma, peripheral T cell lymphoma (unspecified), anaplastic large cell lymphoma, or multiple myeloma (plasma cell myeloma or Kahler's disease).

**In the sixth aspect,** the present invention provides a method for treating a proliferative disease, comprising step of: administering to a subject in need thereof a therapeutically effective amount of the solid dispersion of the first aspect of the present invention, the pharmaceutical composition of the third aspect of the present invention, or the pharmaceutical formulation of the fourth aspect of the present invention.

In some preferred embodiments, the subject is a mammal, such as human.

### BRIEF DESCRIPTION OF THE DRAWINGS

Figure 1 is the PLM image of the amorphous form of API.
Figure 2 is the mDSC pattern of the amorphous form of API.
Figure 3 is the TGA pattern of the amorphous form of API.
Figure 4 is the XRPD pattern of the amorphous form of API.
Figure 5 is the stacked XRPD pattern of the rapid solvent evaporation product.
Figure 6 is the solubility curve of the rapid evaporation product.
Figure 7 is the PLM image of the solid dispersion with the formulation of API+Soluplus.
Figure 8 is the XRPD pattern of the solid dispersion with the formulation of API+Soluplus.
Figure 9 is the TGA pattern of the solid dispersion with the formulation of API+Soluplus.
Figure 10 is the mDSC pattern of the solid dispersion with the formulation of API+Soluplus.
Figure 11 is the PLM image of the solid dispersion with the formulation of API+PVP VA64.
Figure 12 is the XRPD pattern of the solid dispersion with the formulation of API+PVP VA64.
Figure 13 is the TGA pattern of the solid dispersion with the formulation of API+PVP VA64.
Figure 14 is the mDSC pattern of the solid dispersion with the formulation of API+PVP VA64.
Figure 15 is the PLM image of the solid dispersion with the formulation of API+HPC SSL.
Figure 16 is the XRPD pattern of the solid dispersion with the formulation of API+HPC SSL.
Figure 17 is the TGA pattern of the solid dispersion with the formulation of API+HPC SSL.
Figure 18 is the mDSC pattern of the solid dispersion with the formulation of API+HPC SSL.
Figure 19 is the redispersion solubility curves of solid dispersions with different formulations.
Figure 20 is the XRPD pattern of solid dispersions with different formulations under 25 °C/60% RH (open) for one week.
Figure 21 is the XRPD pattern of solid dispersions with different formulations under 40 °C/75% RH (open) for one week.
Figure 22 is the PK profiles of three solid dispersions (formulation of API+Soluplus, formulation of API+PVP VA64, and formulation of API+HPC SSL).
Figure 23 is the PK profiles of different drug loading of the solid dispersion with the formulation of API+HPC SSL.
Figure 24 is the PLM image of the solid dispersion with the formulation of API+HPC SSL (3:7, w/w).
Figure 25 is the XRPD pattern of the solid dispersion with the formulation of API+HPC SSL (3:7, w/w).
Figure 26 is the TGA pattern of the solid dispersion with the formulation of API+HPC SSL (3:7, w/w).
Figure 27 is the mDSC pattern of the solid dispersion of formulation API+HPC SSL (3:7, w/w).
Figure 28 is the PLM image of the solid dispersion with the formulation of API+HPC SSL (4:6, w/w).
Figure 29 is the XRPD pattern of the solid dispersion with the formulation of API+HPC SSL (4:6, w/w).
Figure 30 is the TGA pattern of the solid dispersion with the formulation of API+HPC SSL (4:6, w/w).
Figure 31 is the mDSC pattern of the solid dispersion with the formulation of API+HPC SSL (4:6, w/w).
Figure 32 is the PLM image of the solid dispersion with the formulation of API+HPC SSL (1:1, w/w).
Figure 33 is the XRPD pattern of the solid dispersion with the formulation of API+HPC SSL (1:1, w/w).
Figure 34 is the TGA pattern of the solid dispersion with the formulation of API+HPC SSL (1:1, w/w).
Figure 35 is the mDSC pattern of the solid dispersion with the formulation of API+HPC SSL (1:1, w/w).
Figure 36 is the redispersion solubility curves of the formulation of API+HPC SSL.
Figure 37 is the XRPD pattern of the solid dispersion with the formulation of API+HPC SSL under 25 °C/60% RH (open) for 4 weeks.
Figure 38 is the XRPD pattern of the solid dispersion with the formulation of API+HPC SSL under 40 °C/75% RH (open) for 4 weeks.
Figure 39 is the XRPD pattern of crystalline form G of API.
Figure 40 is the DSC pattern of crystalline form G of API.
Figure 41 is the TGA pattern of crystalline form G of API.
Figure 42-1 and Figure 42-2 are XRPD patterns of the solid dispersion prepared using crystalline form G of API as raw material.
Figure 43-1 and Figure 43-2 are DSC patterns of the solid dispersion prepared using crystalline form G of API as raw material.
Figures 44-1 and 44-2 are TGA patterns of the solid dispersion prepared using crystalline form G of API as raw material.

### DETAILED DESCRIPTION

The inventors developed a solid dispersion with excellent pharmacokinetic characteristics after extensive and intensive research. The solid dispersion of the present invention exhibits a higher plasma exposure in rats, i.e., a higher peak drug concentration and a larger area under the curve, and the area under the curve thereof is 1.5 to 7 times (preferably 5 to 6 times) the area under the curve when the active ingredient is administered alone. On this basis, the present invention was completed.

### Active ingredients

As used herein, the term "active pharmaceutical ingredient (API, also referred to herein as "active ingredient")" refers to one or more selected from the compound of formula A, or a derivative, a crystalline form, an amorphous form, or a pharmaceutically acceptable salt, a hydrate or a solvate thereof as an active ingredient, wherein the chemical name of the compound of formula A is *N*-((*S*)-(5-chloropyridin-2-yl)(cyclobutyl)methyl)-2-((*S*)-2,6-dioxopiperidin-3-yl)-1-oxois oindoline-5-carboxamide.

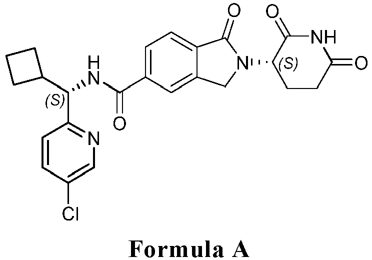

### Solid dispersion

Solid dispersion refers to a dispersed system in solid form formed by highly dispersing the active ingredient in a (solid) carrier.

In the present invention, the solid dispersion includes solid solutions, glass solutions, glass suspensions, amorphous precipitations in a crystalline carrier, eutectic mixtures or monotectics, mixed or composite formations, and combinations thereof.

### Preparation method of solid dispersion

The solid dispersion of the present invention may be prepared by the following methods. However, the conditions of the method, such as carrier, solvent, amount of each component, preparation temperature, preparation time, etc. are not limited to the following explanation. The solid dispersion of the present invention may optionally be conveniently prepared by combining various synthesis methods described in the specification or known in the art, and such combination may be easily performed by those skilled in the art to which the present invention belongs.

The preparation method of the present invention can adopt, for example, hot melt extrusion, hot melt coating, granulation, congelation, solvent evaporation methods (such as layering, coating, and granulation).
preferably, the solid dispersion of the present invention is prepared by the following method:
method 1
mixing one or more of the compound of formula A or a derivative, a crystalline form, an amorphous form thereof, or a pharmaceutically acceptable salt, a hydrate, or a solvate thereof, optionally with other components (such as the carrier of the present invention, wherein the carrier is selected from: one or more of a homopolymer and copolymer of N-vinyl lactam, a cellulose derivative, a graft copolymer, a polyalkylene oxide with a high molecular weight, a polyacrylate and polymethacrylate, a polyacrylamide, a polyvinyl acetate, an oligosaccharide or a polysaccharide or a copolymer thereof, an acrylic acid copolymer) with a solvent (such as water, ethanol, methanol, ethyl acetate, acetone, dichloromethane, acetonitrile, tetrahydrofuran, or a combination thereof) to form a solution or suspension, and removing the solvent to obtain the solid dispersion;
method 2
mixing one or more of the compound of formula A or a derivative, crystalline form, amorphous form thereof, or pharmaceutically acceptable salt, hydrate, or solvate thereof, optionally with other components (such as including the carrier as described above), heating and then extruding to obtain the solid dispersion;
method 3
mixing one or more of the compound of formula A or a derivative, crystalline form, amorphous form thereof, or pharmaceutically acceptable salt, hydrate, or solvate thereof, optionally with other components (such as including the carrier as described above), with a solvent (such as water, ethanol, methanol, ethyl acetate, acetone, dichloromethane, acetonitrile, tetrahydrofuran, or a combination thereof), and spray-drying (the inlet temperature of air is set to 40 °C ~ 200 °C, preferably 80 °C ~ 120 °C) to obtain the solid dispersion.

Preferably, the weight to volume ratio of one or more of the compound of formula A or a derivative, crystalline form, amorphous form thereof, or pharmaceutically acceptable salt, hydrate, or solvate thereof to the solvent is (0.1~30):1 mg/mL, preferably (1~10):1 mg/mL, and more preferably 5:1.5 mg/mL;
and/or, the solvent is an alcohol solvent and/or a halogenated hydrocarbon solvent, wherein the alcohol solvent is preferably methanol or ethanol; the halogenated hydrocarbon solvent is preferably dichloromethane; preferably, the solvent is dichloromethane and methanol, or, dichloromethane and ethanol, wherein the volume ratio of dichloromethane to methanol, or, dichloromethane to ethanol is preferably 9:1~1:1;
and/or, the solvent is acetone and water, wherein the volume ratio of acetone to water is preferably 7:1~10:1.

### Pharmaceutical composition

The pharmaceutical composition of the present invention may be prepared by various methods well known in the art, which may be prepared by mixing a therapeutically effective amount of the solid dispersion with one or more pharmaceutically acceptable excipients into a formulation suitable for human administration, such as the above-mentioned tablet, capsule, granule, etc.

"Therapeutically effective amount" refers to the amount of active ingredient according to the present invention, which, when administered to a patient in need thereof, is sufficient to achieve treatment of the disease state, condition or disorder for which the active ingredient has efficacy. Such an amount will be sufficient to elicit the biological or medical response sought by a researcher or clinician in a tissue system or patient.

The pharmaceutical composition of the present invention comprises a safe and effective amount of the solid dispersion of the present invention or a pharmacologically acceptable salt thereof and a pharmacologically acceptable excipient. Wherein "safe and effective amount" means: the amount of the solid dispersion is sufficient to significantly improve the condition without causing serious side effects. Usually, the pharmaceutical composition contains 1 ~ 2000 mg/dose of the solid dispersion of the present invention, and more preferably, contains 10 ~ 200 mg/dose of the solid dispersion of the present invention. Preferably, the "one dose" is one capsule or tablet.

Excipients refer to one or more compatible solid or liquid fillers or gel substances that are suitable for human use and must have sufficient purity and sufficiently low toxicity. "Compatibility" used herein means that the components in the composition may be mixed with the solid dispersion of the present invention and with each other without significantly reducing the efficacy of the solid dispersion. Some examples of pharmaceutically acceptable excipients include cellulose and derivatives thereof (such as sodium carboxymethyl cellulose, sodium ethyl cellulose, cellulose acetate, etc.), starches, monosaccharides or polysaccharides, gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agents (such as sodium lauryl sulfate), colorants, flavoring agents, stabilizers, antioxidants, preservatives, pyrogen-free water, etc.

There is no particular limitation to the administration methods of the solid dispersion or pharmaceutical composition of the present invention. Representative administration methods include (but are not limited to): oral, intratumoral, rectal, parenteral (intravenous, intramuscular or subcutaneous), and topical administration.

Solid formulations for oral administration include capsules, tablets, pills, powders and granules. In these solid formulations, the solid dispersion is mixed with at least one conventional inert excipient (or carrier), such as sodium citrate or dicalcium phosphate, or with the following ingredients: (a) fillers or extenders, such as starch, lactose, sucrose, glucose, microcrystalline cellulose, mannitol and silicic acid; (b) binders, such as hydroxypropyl methylcellulose, alginates, gelatin, polyvinyl pyrrolidone, sucrose and gum arabic; (c) humectants, such as glycerol; (d) disintegrants, such as agar, calcium carbonate, potato starch or tapioca starch, alginic acid, certain complex silicates, cross-linked sodium carboxymethylcellulose and sodium carbonate; (e) solubilizers, such as paraffin; (f) absorption accelerators, such as quaternary ammonium compounds; (g) wetting agents, such as cetyl alcohol, sodium lauryl sulfate, glyceryl monostearate; (h) adsorbents, such as kaolin; and (i) lubricants, for example, talc, calcium stearate, magnesium stearate, solid polyethylene glycol, sodium lauryl sulfate, or mixtures thereof. In capsules, tablets and pills, the formulation may further contain a buffer.

Solid formulations such as tablets, sugar pills, capsules, pills and granules may be prepared using coatings and shell materials, such as enteric coatings and other materials known in the art. They may contain opacifiers, and the release of the solid dispersion in such compositions may be delayed in a certain part of the digestive tract. Examples of embedding components that may be used are polymeric substances and waxes. If necessary, the solid dispersion can also be formed into microcapsules with one or more of the above-mentioned excipients.

Liquid formulations for oral administration include pharmaceutically acceptable emulsions, solutions, suspensions, syrups or tinctures. In addition to solid dispersions, liquid formulations may contain inert diluents conventionally used in the art, such as water or other solvent, solubilizers and emulsifiers, for example, ethanol, isopropanol, ethyl carbonate, ethyl acetate, propylene glycol, 1,3-butylene glycol, dimethylformamide and oils, in particular cottonseed oil, peanut oil, corn germ oil, olive oil, castor oil and sesame oil or mixtures of these substances, etc.

In addition to these inert diluents, the composition may further comprise adjuvants such as wetting agents, emulsifying and suspending agents, sweeteners, flavoring agents, and perfumes.

In addition to solid dispersions, suspensions may comprise suspending agents such as, for example, ethoxylated isostearyl alcohols, polyoxyethylene sorbitol and sorbitan esters, microcrystalline cellulose, aluminum methanol and agar, or mixtures of these substances, etc.

Compositions for parenteral injection may comprise physiologically acceptable sterile aqueous or anhydrous solutions, dispersions, suspensions or emulsions, and sterile powders for reconstitution into sterile injectable solutions or dispersions. Suitable aqueous and non-aqueous carriers, diluents, solvent or excipients comprise water, ethanol, polyols and suitable mixtures thereof.

The formulations of the solid dispersion of the present invention for topical administration comprise ointments, powders, patches, sprays and inhalants. The solid dispersion of the present invention is mixed under sterile conditions with a physiologically acceptable carrier and any preservatives, buffers, or propellants that may be required if necessary.

The solid dispersion of the present invention may be administered alone or in combination with other pharmaceutically acceptable compounds. When using a pharmaceutical composition, a safe and effective amount of the solid dispersion of the present invention is applied to a mammal (such as a human) in need of treatment, wherein the dosage during administration is a pharmaceutically effective dosage. For a person weighing 60 kg, the daily dosage is usually 0.2 ~ 2000 mg, preferably 1 ~ 500 mg, and more preferably 1 ~ 200 mg. Of course, the specific dosage should also take into account factors such as the route of administration and the patient's health conditions, which are all within the range of skilled physicians.

The specific carriers mentioned in the present invention, such as PVP K30, PVP VA64, HPMCAS 716G, HPMCAS 912G, HPMCAS 126G, HPMCAS 716F, HPMCAS 912F, HPMCAS 126F, HPMCAS LG, HPMCAS MG, HPMCAS HG, HPMCAS LF, HPMCAS MF, HPMCAS HF, HPC SSL, Eudragit L100, Eudragit E100, Eudragit S100, Eudragit L100-55, Eudragit EPO, Soluplus, etc. are all commercially available products and have disclosed structural compositions and/or properties. Detailed information such as specific structures and properties is available in the relevant product instructions.

The solid dispersion, pharmaceutical composition or pharmaceutical formulation provided by the present invention has acceptable biological absorption performance. Such biological absorption may be demonstrated by pharmacokinetic (PK) properties, more particularly by Cmax or AUC at a specific dose or within a dose range. In addition, bioavailability may be determined by PK studies in humans or in any suitable model species. The solid dispersion of the present invention can significantly improve the oral bioavailability of the compound of formula A and has good solid stability.

The present invention is further described below by way of examples, but the present invention is not limited to the scope of the examples. The experimental methods in the following examples without indicating specific conditions are carried out according to conventional methods and conditions, or selected according to the product specifications.

In the following examples, all reagents and materials used are commercially available or purchased from the following suppliers.

| **Name** | **Level** | **Suppliers** |
|---|---|---|
| TFA | HPLC | J&K Scientific |
| Acetonitrile (ACN) | HPLC | Merck |
| MeOH | HPLC | Merck |
| EtOH | N/A | STA-DFR |
| SIF powder | N/A | Biorelevant |
| PVP K30 | N/A | BASF |
| PVP VA64 | N/A | BASF |
| HPC SSL | N/A | Tsao Da Co., LTD |
| Soluplus | N/A | BASF |
| Acetone | CMW | SIGMA-ALDRICH |
| DCM | HPLC | SIGMA-ALDRICH |
| DCM | N/A | STA-DFR |
| Water | N/A | STA-DFR |
| Ethyl acetate (EA) | N/A | SIGMA-ALDRICH |
| HPMC E3 | N/A | Shanghai Colorcon Coating Technology Co., Ltd. |
| HPMCAS-MG | N/A | Shinetsu |
| HPMCAS-HG | N/A | Shinetsu |
| Eudragit L100 | N/A | EVONIK |
| PG | 98% | Beijing Feilongrui Trading Co., Ltd. |
| Isopropyl alcohol | N/A | SIGMA-ALDRICH |
| N-butyl alcohol | AR | Shanghai Experimental Reagent Co., Ltd. |
| n-propyl alcohol | AR | Shanghai Experimental Reagent Co., Ltd. |
| THF | HPLC | T.T.Baker |

### Abbreviation

| **Abbreviation** | **Full Name** |
|---|---|
| ACN | Acetonitrile |
| API | Active pharmaceutical ingredient |
| DSC | Differential Scanning Calorimetry |
| DVS | Dynamic Vapor Sorption |
| DCM | Dichloromethane |
| DMSO | Dimethyl sulfoxide |
| DMAc | N,N-Dimethylacetamide |
| EtOH | Ethanol |
| EA | Ethyl acetate |
| Acetone | Acetone |
| HPLC | High Performance Liquid Chromatography |
| hr | Hour |
| KF | Karl Fischer |
| 2-MeTHF | 2-Methyltetrahydrofuran |
| MeOH | Methanol |
| MTBE | Methyl tert-butyl ether |
| NMR | Nuclear Magnetic Resonance |
| NMM | N-Methylmorpholine |
| TGA | Thermogravimetric analysis |
| THF | Tetrahydrofuran |
| T₃P | Propyl phosphoric anhydride |
| IPA | Isopropyl acetate |
| IPA | Isopropyl alcohol |
| PLM | Polarized Light Microscopy |
| PK | Pharmacokinetics |
| onset | Initial value |
| peak | Peak |
| XRPD | X-ray Powder Diffraction |
| RH | Relative Tumidity |
| LOQ | Limit of Quantitation |
| RRT | Relative Retention Time |

### Measurement method of Polarized Light Microscopy (PLM):

- Nikon LV100POL is equipped with a 5-megapixel CCD
- Physical lens: 10x ~ 50x

### X-ray Powder Diffractometer (XRPD) determination method:

The sample is performed on XRPD using the following method:
- Tube: Cu:K-α (λ=1.54179Ǻ)
- Generator: voltage: 40 kV; current: 40 mA
- Scanning range: 3 to 40 degrees
- Sample speed: 15 rpm
- Scanning speed: 10 degrees/minute

### Measurement of Thermogravimetric Analysis (TGA):

- The sample (2~5 mg) was put on an aluminum pan and performed as follows:
- The sample was heated from room temperature to 300 °C at a rate of 10 °C /min under atmospheric conditions. If the weight loss of the sample exceeds 20%, the test is completed.

### Measurement of Differential Scanning Calorimetry (DSC):

- The sample (~1 mg) was tested using a sealed aluminum pan with a pinhole
- The sample was heated from 30 °C to 220 °C at a rate of 10 °C /min.

### Preparation of the compound of formula A

The compound of formula A was prepared according to the method of Example 15 of PCT/CN2022/097236, and its chemical name is *N*-((*S*)-(5-chloropyridin-2-yl)(cyclobutyl)methyl)-2-((*S*)-2,6-dioxopiperidin-3-yl)-1-oxoisoind oline-5-carboxamide.

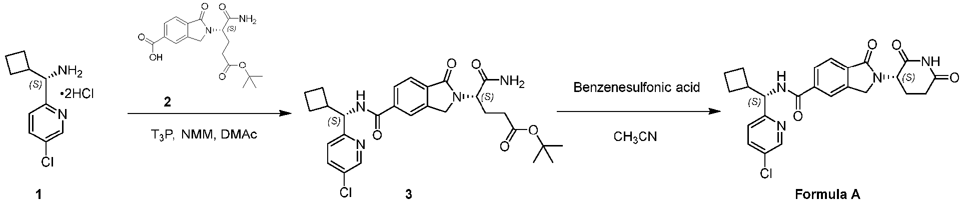

Compound 1 (3.25 g, 12.3 mmol, hydrochloride), compound 2 (4.67 g, 12.9 mmol), NMM (6.2 g, 61.3 mmol) and T₃P (5.6 g, 17.6 mmol) were added to a round-bottom flask containing DMAc (50 mL). The mixture was degassed and purged with N₂ for 3 times. The mixture was stirred at 25 °C under N₂ for 12 hours. The mixture was poured into a saturated aqueous sodium chloride solution (100 mL), then filtered and washed with water (100 mL). The filter cake was then dissolved in DCM (200 mL), washed with a saturated aqueous NaHCO₃ solution (100 mL), and the organic phase was dried with anhydrous Na₂SO₄, filtered and concentrated under reduced pressure to obtain compound 3 (6.5 g, 99.7% yield based on compound 1) as a white solid.

Benzenesulfonic acid (1.1 g, 7.21 mmol) was added to a solution of compound 3 in CH₃CN (13 mL). The mixture was stirred at 70 °C for 14 hours under N₂ atmosphere, the mixture was diluted with DCM (60 mL), washed with saturated NaHCO₃ aqueous solution (30 mL x 2) and then washed with H₂O (30 mL x 2). The organic phase was dried with anhydrous Na₂SO₄, filtered and concentrated under reduced pressure to obtain a crude product, which was slurried with EA (5 mL) and MTBE (5 mL) and filtered to obtain a filter cake of compound A, which was defined as API control after drying. The API control filter cake was added to 30 mL of acetonitrile, ultrasonically treated for 5 minutes to uniformly disperse it into a slurry solution, and then 70 mL of water was added to make the entire solution clear and transparent. After lyophilization, an amorphous off-white solid of compound A was obtained, which was defined as amorphous form of API, with a yield of 87% and a purity of 99%.

¹H NMR (400 MHz, DMSO-*d₆*) δ11.01 (s, 1H), 8.91 (d, *J* = 8.0 Hz, 1H), 8.57 - 8.52 (m, 1H),8.06 (s, 1H), 8.02 - 7.95 (m, 1H), 7.91 - 7.87 (m, 1H), 7.81 (d, *J =* 8.0 Hz, 1H), 7.49 (d, *J =* 8.4 Hz, 1H), 5.19 - 5.05 (m, 2H), 4.57 - 4.34 (m, 2H), 2.97 - 2.81 (m, 2H), 2.65 - 2.56 (m, 1H), 2.45 - 2.35 (m, 1H), 2.12 - 1.97 (m, 2H), 1.91 - 1.71 (m, 5H); LCMS (ESI+): m/z 467.2 [M+H]+.

### Example 1 Amorphous characteristics of the compound of formula A (active pharmaceutical ingredient, API)

### PLM, XRPD, mDSC and TGA were used for characterization.

As shown in Figure 1 and Figure 4, PLM and XRPD show that it is amorphous, and the diffraction peaks (characteristic peaks) of the compound of formula A all disappeared, and it was an amorphous or molecular form. As shown in Figure 3, it had a weight loss of 2.79 wt% before 210 ± 2.0 °C. As shown in Figure 4, mDSC shows a glass transition temperature of 129.30 ± 2.0 °C.

### Example 2 Approximate solubility test

About 5 mg of the API control of Formula A was weighed into a 1.5 mL glass vial, and then a certain amount of different solvents (as shown in Table 1) were gradually added until the solid dissolved or the total volume reached 1 mL. The compound of formula A has a high approximate solubility in a mixed solvent of DCM and MeOH. However, in other studies, it was found that the compound of formula A rapidly racemized in MeOH; in acetone and ethanol, the compound of formula A has better chirality stability.

The approximate solubility results are shown in Table 1. Different solvents have different solubility for the compound of formula A, considering the stability factor, DCM:EtOH (9:1, v/v) and acetone:water (9:1, v/v) may be used as solvent systems for the preparation of solid dispersions of the compound of formula A.

**Table 1 Approximate solubility of the compound of formula A**

| NO. | Solvents | Solubility (mg/mL) | NO. | Solvents | Solubility (mg/mL) |
|---|---|---|---|---|---|
| 1 | MeOH | S<5 | 12 | DCM:THF (1:1) | S<5 |
| 2 | Acetone | S<5 | 13 | DCM:MeOH (1:1) | 25<S<50 |
| 3 | DCM | S<5 | 14 | DCM:MeOH (2:1) | 50<S<100 |
| 4 | THF | S<5 | 15 | DCM:MeOH (4:1) | 50<S<100 |
| 5 | ACN | S<5 | 16 | DCM:MeOH (5:1) | 50<S<100 |
| 6 | Ethyl acetate | S<5 | 17 | DCM:MeOH (7:1) | 50<S<100 |
| 7 | Acetone:DCM (4:1) | S<5 | 18 | DCM:MeOH (9:1) | 25<S<50 |
| 8 | Acetone:DCM (1:1) | S<5 | 19 | DCM:MeOH (99:1) | S<5 |
| 9 | Acetone:THF (1:1) | S<5 | 20 | DCM:EtOH (9:1) | 5<S<15 |
| 10 | Acetone:ACN (1:1) | S<5 | 21 | Acetone:Water (9:1) | 15 <S< 50 |
| 11 | DCM:THF (4:1) | S<5 | / | / | / |

### Example 3 Rapid solvent evaporation test

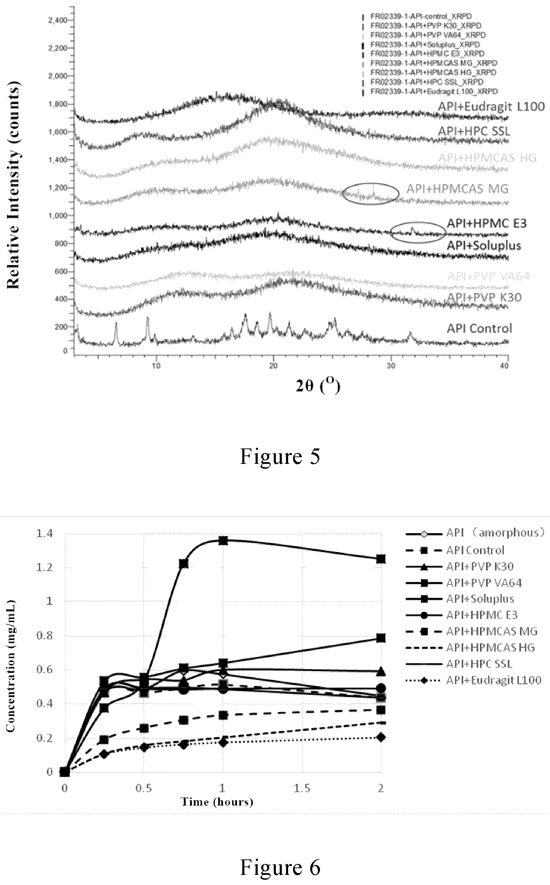

About 10 mg of the API control of the compound of formula A and 40 mg of the polymer carrier were transferred to 1 mL of DCM: EtOH = 9:1 (v/v) to prepare a solution with 20% drug loading. The resulting clear solution was rapidly evaporated at 80 °C until there was no liquid to prepare a solid dispersion. The API control of the compound of formula A was also treated separately in the same manner. As shown in Figure 5, two products (API+HPMC E3 and API+HMCAS MG) had crystalline diffraction peaks, and among the remaining samples, except for the API control of the compound of formula A, all other rapid solvent evaporation products were amorphous.

Furthermore, as shown in Table 2 and Figure 6, the solubility of the evaporated product at different time points was measured in simulated fasting intestinal fluid (FaSSIF) to evaluate the effects of different types of polymers on the solubility of the compound of formula A. Compared with the amorphous form of API and the API control, the products using PVP K30, PVP VA64 and Soluplus as carriers showed relatively great improvement in the solubility of the compound of formula A.

Based on the results of the inhibitory effect of polymer carriers on API crystal form (crystal diffraction peaks) and solubility improvement performance, three polymers, PVP VA64, Soluplus and HPC SSL, were selected for further investigation.

**Table 2 Solubility results of rapid evaporation products in FaSSIF**

| NO. | Formulations | Solubility (mg/mL) | | | | |
|---|---|---|---|---|---|---|
| | | 0.25 h | 0.5 h | 0.75 h | 1 h | 2 h |
| 0 | Amorphous form of API | 0.47 | 0.49 | 0.60 | 0.57 | 0.45 |
| 1 | API control | 0.47 | 0.46 | 0.49 | 0.51 | 0.44 |
| 2 | API+PVP K30 | 0.47 | 0.54 | 0.54 | 0.60 | 0.59 |
| 3 | API+PVP VA64 | 0.53 | 0.56 | 0.61 | 0.64 | 0.78 |
| 4 | API+Soluplus | 0.37 | 0.53 | 1.22 | 1.36 | 1.25 |
| 5 | API+HPMC E3 | 0.49 | 0.49 | 0.48 | 0.49 | 0.49 |
| 6 | API+HPMCAS MG | 0.19 | 0.26 | 0.30 | 0.33 | 0.36 |
| 7 | API+HPMCAS HG | 0.11 | 0.16 | 0.18 | 0.20 | 0.29 |
| 8 | API+HPC SSL | 0.48 | 0.49 | 0.49 | 0.48 | 0.43 |
| 9 | API+Eudragit L100 | 0.11 | 0.14 | 0.16 | 0.17 | 0.20 |

### Example 4 Preparation of solid dispersions by spray-drying

Spray-drying parameter setting: the spray-drying parameters were set according to Table 3.

**Table 3 Spray-drying parameters of solid dispersions**

| Parameter settings | Formulation of API+Soluplus (1:4, *w*/*w*) | Formulation of API+PVP VA64 (1:4, *w*/*w*) | Formulation of API+HPC SSL (1:4, *w*/*w*) |
|---|---|---|---|
| instrument | Buchi B290 | | |
| Solvents | DCM: EtOH (9:1, *v*/*v*) | | |
| API concentration (mg/mL) | 10 | | |
| inlet temperature (°C) | 90 | | |
| Pump speed % | 30 | | |
| Inhalation % | 100 | | |
| Flow | 35 | | |
| Actual inlet temperature (°C) | 87-92 | 85-90 | 86-90 |
| Outlet (°C) | 55-57 | 54-56 | 55-58 |

Based on the results of the rapid solvent evaporation test, three polymer carriers, including PVP VA64, Soluplus and HPC SSL, were selected to prepare solid dispersions of the compound of formula A by a spray-drying method.

As shown in Table 3, according to the ratios of formulations, an appropriate amount of the API control of the compound of formula A and the corresponding polymer carriers were weighed, and then DCM:EtOH (9:1, v/v) solvent was added (DCM was first added to wet the API, and then EtOH was added). All samples were then stirred at 700 rpm until completely dissolved, and the sample solution was spray-dried according to the equipment parameters in Table 3. The obtained solid powder was further dried overnight (about 22 hours) under vacuum conditions at 40 °C to obtain a solid dispersion sample.

Table 4 shows the characterization results of solid dispersions with different formulations by PLM, XRPD, mDSC, TGA and HPLC.

**Table 4 Summary of properties of solid dispersions with different carriers**

| Formulations | | Formulation of API+Soluplus (1:4, *w*/*w*) | Formulation of API+PVP VA64 (1:4, *w*/*w*) | Formulation of API+HPC SSL (1:4, *w*/*w*) |
|---|---|---|---|---|
| Target drug loading (%) | | 20% | | |
| Appearance | | White powder | | |
| PLM | | Irregular block & no birefringence | | |
| XRPD | | Amorphous | | |
| Tg of mDSC (°C) | | 99.79 | 118.74 | 70.72 |
| TGA weight loss (%) | | 0.96 | 1.90 | 1.00 |
| HPLC | Drug loading, % | 20.54 | 20.5 | 20.56 |
| | Purity, % | 99.32 | 99.35 | 99.29 |
| | Content, % | 103.15 | 102.95 | 103.30 |
| Yield (%) | | 79.1 | 83.6 | 66.3 |

The solid dispersion with the formulation of API+Soluplus (1:4, w/w) was characterized by PLM, XRPD, mDSC and TGA. As shown in Table 4 and Figures 7 to 10, PLM and XRPD show that it was amorphous, and the XRPD diffraction peaks (characteristic peaks) of the API in the solid dispersion all disappeared, present in an amorphous form. As shown in Figure 9, the weight loss was 0.96% at 180 ± 2.0 °C. As shown in Figure 10, mDSC showed a glass transition temperature of 99.79 ± 2.0 °C. The content result tested by HPLC was 103.15%, and the purity did not change significantly, about 99.32%.

The solid dispersion with the formulation of API+PVP VA64 (1:4, w/w) was characterized by PLM, XRPD, mDSC and TGA. As shown in Table 4 and Figures 11 to 14, PLM and XRPD show that it was amorphous, and the XRPD diffraction peaks (characteristic peaks) of the API in the solid dispersion all disappeared, present in an amorphous form. As shown in Figure 13, the weight loss was 1.90% at 210 ± 2.0 °C. As shown in Figure 14, mDSC showed a glass transition temperature of 118.74 ± 2.0 °C. The content result tested by HPLC was 102.95%, and the purity did not change significantly, about 99.35%.

The solid dispersion with the formulation of API+HPC SSL (1:4, w/w) was characterized by PLM, XRPD, mDSC and TGA. As shown in Table 4 and Figures 15 to 18, PLM and XRPD show that it was amorphous, and the XRPD diffraction peaks (characteristic peaks) of the API in the solid dispersion all disappeared, present in an amorphous form. As shown in Figure 17, the weight loss was 1.00% at 180 ± 2.0 °C. As shown in Figure 18, mDSC showed a glass transition temperature of 70.72 ± 2.0 °C. The content result tested by HPLC was 103.30%, and the purity did not change significantly, about 99.29%.

### Example 5 Redispersion Solubility test of the solid dispersions

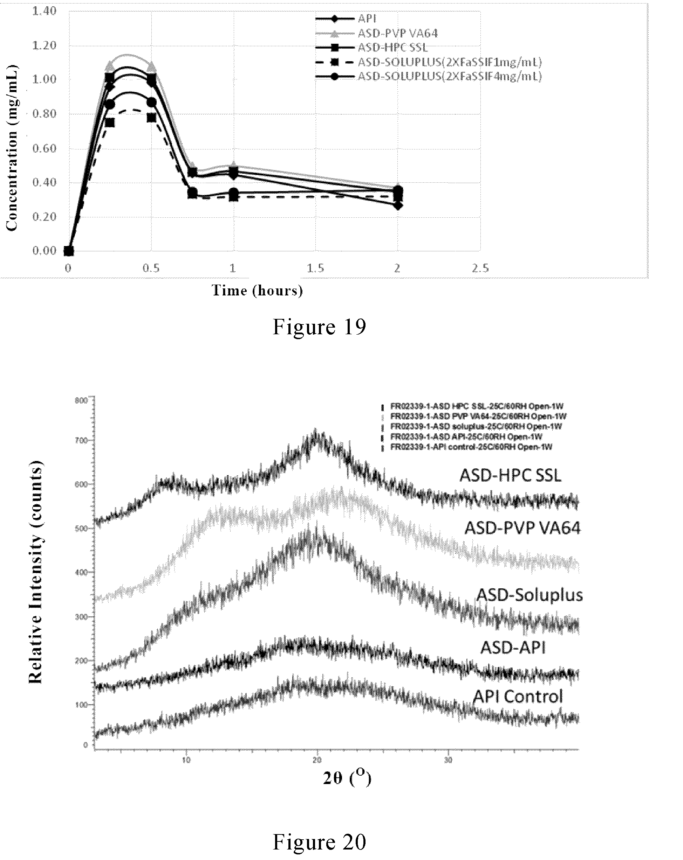

Method: the formulation sample powders shown in Table 5 were weighed into 3 mL of simulated gastric fluid (SGF) with a target API concentration as 8 mg/mL or 2 mg/mL. After stirring at 37 °C for 0.25 h and 0.5 h at 500 rpm, about 200 µL of the suspension was centrifuged at 14,000 rpm for 5 min, and the supernatant was diluted by 6 times with diluent for HPLC analysis. After sampling at 0.5 h, 2 times the volume of SGF of FaSSIF was immediately added to the suspension and the mixture was stirred for another 0.25 h, 0.5 h and 1.5 h, and then sampled for HPLC analysis and pH testing. The results are shown in Table 5 and Figure 19. The formulation of API + PVP VA64 and the formulation of API + HPC SSL showed better solubility.

**Table 5. Test results of redispersion solubility of the solid dispersion of the compound of formula A**

| Formulations | Concentrations (mg/mL) | Step 1: SGF | | Step 2: SGF + 2 times volume FaSSIF | | | End point pH |
|---|---|---|---|---|---|---|---|
| | | (0.25 hr) | (0.5 hr) | (0.75 hr) | (1.0 hr) | (2.0 hr) | |
| Amorphous form of API | 1 mg/mL | 0.96 | 0.98 | 0.46 | 0.44 | 0.27 | 6.55 |
| API+Soluplus | 1 mg/mL | 0.75 | 0.78 | 0.33 | 0.31 | 0.32 | 6.57 |
| API+Soluplus | 4 mg/mL | 0.86 | 0.87 | 0.34 | 0.34 | 0.35 | 6.56 |
| API+PVP VA64 | 1 mg/mL | 1.08 | 1.08 | 0.49 | 0.50 | 0.37 | 6.57 |
| API+HPC SSL | 1 mg/mL | 1.01 | 1.01 | 0.46 | 0.46 | 0.35 | 6.57 |

### Example 6 One-week solid stability study of solid dispersions

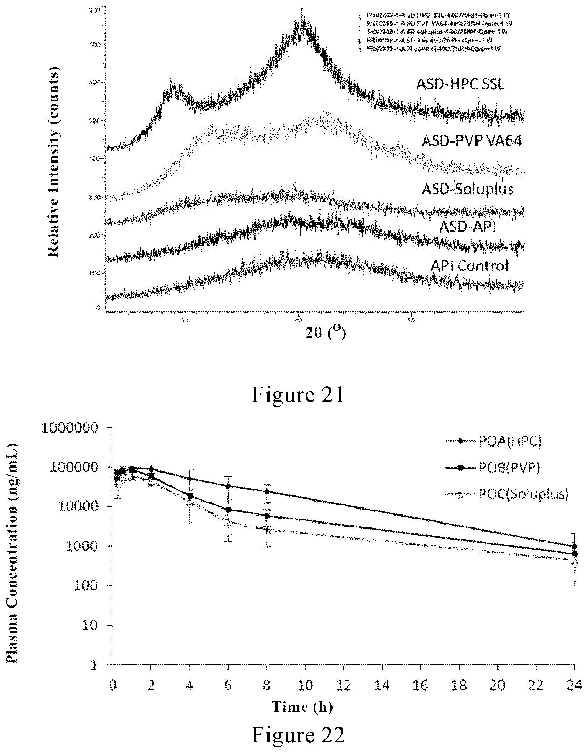

According to Table 6, three solid dispersions, namely, formulation of API+Soluplus, formulation of API+PVP VA64, and formulation of API+HPC SSL, were stored at 25 °C /60%RH (open) and 40 °C/75%RH (open) for one week, the appearance of the sample was visually observed and the purity of the samples was analyzed by HPLC. The appearance results are shown in Table 7. There was slight agglomeration and transparent gel under 40 °C /75%RH (open). XRPD characterization is shown in Figure 20 and Figure 21.

The results showed that the chemical stability of the three solid dispersions, namely, formulation of API+Soluplus, formulation of API+PVP VA64, and formulation of API+HPC SSL, is relatively poor at 40 °C /75%RH (open), as shown in the HPLC analysis results in Table 6. However, the three solid dispersions all showed good physical stability under the same conditions. Therefore, the storage of solid dispersions should avoid exposure to high temperature and high humidity conditions to prevent chemical degradation.

**Table 6 HPLC results of one-week solid stability of solid dispersions**

| Samples | Conditions | Impuritie s % | Conte nts % | RRT (%) | | | | | | | | |
|---|---|---|---|---|---|---|---|---|---|---|---|---|
| | | | | 0.54 | 0.92 | 0.93 | 0.95 | 0.96 | 1.00 | 1.11 | 1.18 | 1.24 |
| API control | Initial | 0.67 | / | 0.11 | <LOQ | 0.09 | 0.09 | 0.10 | 99.3 4 | 0.07 | 0.15 | 0.05 |
| | 25 °C/60%RH open | 0.72 | 97.26 | 0.10 | <LOQ | 0.10 | 0.09 | 0.10 | 99.2 9 | 0.09 | 0.17 | 0.06 |
| | 40 °C/75%RH open | 1.05 | 96.79 | 0.13 | 0.12 | 0.26 | 0.09 | 0.13 | 98.9 5 | 0.11 | 0.14 | 0.06 |
| API+ Soluplus | Initial | 0.68 | 103.2 | 0.12 | <LOQ | 0.09 | 0.08 | 0.09 | 99.3 2 | 0.08 | 0.15 | 0.07 |
| | 25 °C/60%RH open | 0.72 | 99.82 | 0.10 | <LOQ | 0.07 | 0.10 | 0.11 | 99.2 8 | 0.11 | 0.16 | 0.08 |
| | 40 °C/75%RH open | 1.05 | 99.69 | 0.13 | 0.13 | 0.24 | 0.09 | 0.12 | 98.9 5 | 0.13 | 0.16 | 0.05 |
| API+ PVP VA64 | Initial | 0.65 | 103.0 | 0.10 | <LOQ | 0.08 | 0.09 | 0.11 | 99.3 5 | 0.06 | 0.16 | 0.05 |
| | 25 °C/60%RH open | 0.70 | 100.4 | 0.10 | <LOQ | 0.06 | 0.11 | 0.12 | 99.3 0 | 0.10 | 0.15 | 0.06 |
| | 40 °C/75%RH open | 1.03 | 98.74 | 0.11 | 0.11 | 0.23 | 0.11 | 0.13 | 98.9 7 | 0.11 | 0.16 | 0.07 |
| API+ HPC-SSL | Initial | 0.71 | 103.3 | 0.11 | <LOQ | 0.08 | 0.09 | 0.11 | 99.2 9 | 0.08 | 0.17 | 0.07 |
| | 25 °C/60%RH open | 0.72 | 99.99 | 0.12 | <LOQ | 0.09 | 0.09 | 0.11 | 99.2 9 | 0.09 | 0.15 | 0.07 |
| | 40 °C/75%RH open | 1.49 | 98.08 | 0.10 | 0.29 | 0.60 | 0.08 | 0.10 | 98.5 1 | 0.09 | 0.16 | 0.07 |

**Table 7 Appearance characterization of one-week solid stability of solid dispersion**

| Samples | Initial | 25 °C/60%RH, open | 40 °C/75%RH, open |
|---|---|---|---|
| API control | White powder | White powder | Slight agglomeration |
| API+Soluplus | White powder | White powder | Transparent gel |
| API+ PVP VA64 | White powder | White powder | Slight agglomeration |
| API+HPC-SSL | White powder | White powder | Slight agglomeration |

### Example 7: Drug loading optimization using the API+HPC SSL solid dispersion as an example

According to the results of the studies in Examples 1-6, the drug loading optimization study was conducted using the solid dispersion of API+HPC SSL as an example. As shown in Table 8, an appropriate amount of the API control of Formula A compound and HPC SSL were weighed into the DCM: EtOH (9:1, v/v) solvent, and then all samples were stirred at 700 rpm until completely dissolved, and the sample solution was spray dried according to the equipment parameters shown in Table 8, and the obtained solid powder was further dried under vacuum conditions at 40 °C for about 20 hours to obtain a solid dispersion sample.

### Example 7-1 Preparation of API+HPC SSL solid dispersion with 30-50% drug loading

**Table 8 Spray-drying parameters of API+HPC SSL**

| Parameter settings | Formulation of API+HPC SSL (3:7, *w*/*w*) | Formulation of API+HPC SSL (4:6, *w*/*w*) | Formulation of API+HPC SSL (1:1, *w*/*w*) |
|---|---|---|---|
| Target drug loading (%) | 30 | 40 | 50 |
| instrument | Buchi B290 | | |
| Solvent | DCM: EtOH (9:1, *v*/*v*) | | |
| API concentration (mg/mL) | 10 | | |
| inlet temperature (°C) | 90 | | |
| Pump speed % | 30 | | |
| Inhalation % | 100 | | |
| Flow | 35 | | |
| Actual inlet temperature (°C) | 87-92 | 85-90 | 86-90 |
| Outlet (°C) | 55-57 | 54-56 | 55-58 |

### Example 7-2 Characterization of the solid dispersions with the formulation of API + HPC SSL

The solid dispersions with the formulation of API + HPC SSL shown in Table 9 was characterized by PLM, XRPD, mDSC, TGA and HPLC.

**Table 9 Characteristics of ASD of API+HPC SSL**

| Formulations | | Formulation of API+HPC SSL (3:7, *w*/*w*) | Formulation of API+HPC SSL (4:6, *w*/*w*) | Formulation of API+HPC SSL (1:1, *w*/*w*) |
|---|---|---|---|---|
| Target drug loading (%) | | 30% | 40% | 50% |
| Appearance | | White powder | | |
| PLM | | Irregular block & no birefringence | | |
| XRPD | | Amorphous | | |
| Tg of mDSC (°C) | | 77.43 | 84.97 | 82.54 |
| TGA weight loss (%) | | 0.75 | 1.53 | 1.35 |
| HPLC | Drug loading (%) | 30.98 | 40.34 | 50.28 |
| | Purity (%) | 99.26 | 99.24 | 99.23 |
| | Content (%) | 103.2 | 100.8 | 100.5 |

The solid dispersion with the formulation of API+ HPC SSL (3:7, w/w) was characterized by PLM, XRPD, mDSC and TGA. As shown in Table 9 and Figures 24 to 27, PLM and XRPD showed that the sample was amorphous. No birefringence in PLM, only the halo of XRPD, and the diffraction peaks (characteristic peaks) of API disappeared completely, present in an amorphous form. As shown in Figure 26, the weight loss was 0.75 % before reaching 180 ± 2.0 °C. As shown in Figure 27, mDSC showed a glass transition temperature of 77.43 ± 2.0 °C. The HPLC test results showed no significant change in purity.

The solid dispersion with the formulation of API+ HPC SSL (4:6, w/w) was characterized by PLM, XRPD, mDSC and TGA. As shown in Table 9 and Figures 28 to 31, PLM and XRPD showed that the sample was amorphous. No birefringence in PLM, only the halo of XRPD, and the diffraction peaks (characteristic peaks) of API disappeared completely, present in an amorphous form. As shown in Figure 30, the weight loss was 1.53% before reaching 180 ± 2.0 °C. As shown in Figure 31, mDSC showed a glass transition temperature of 84.97 ± 2.0 °C. The HPLC test results showed no significant change in purity.

The solid dispersion with the formulation of API+ HPC SSL (1:1, w/w) was characterized by PLM, XRPD, mDSC and TGA. As shown in Table 9 and Figures 32 to 35, PLM and XRPD showed that the sample was amorphous. No birefringence in PLM, only the halo of XRPD, and the diffraction peaks (characteristic peaks) of API disappeared completely, present in an amorphous form. As shown in Figure 32, the weight loss was 1.35% before reaching 180 ± 2.0 °C. As shown in Figure 35, mDSC showed a glass transition temperature of 82.54 ± 2.0 °C. The HPLC test results showed no significant change in purity.

### Example 7-3 Redispersion solubility of the solid dispersions with the formulation of API+HPC SSL in biorelevant solvents

The solid dispersion with the formulation of API+HPC SSL with different drug loading were weighed into 3 mL SGF with a target API concentration of 2 mg/mL. After stirred at 500 rpm for 0.25 h and 0.5 h at 37 °C, about 200 µL of the suspension was centrifuged at 14,000 rpm for 5 min and the supernatant was diluted for HPLC analysis. After sampling at 0.5 h, 2 times the volume of SGF FaSSIF was immediately added to the suspension and the mixture was stirred for another 0.25 h, 0.5 h and 1.5 h before sampling for HPLC analysis and pH testing. As shown in Table 10 and Figure 36, three solid dispersions with different drug loading, 30%, 40% and 50% (API:HPC SSL), showed no significant difference in solubility.

**Table 10 Redispersion solubility of API+HPC SSL solid dispersions with different drug loading in biorelevant solvent**

| Formulations | Step 1: SGF | | Step 2: SGF + 2x volume FaSSIF | | |
|---|---|---|---|---|---|
| | 0.25 hr | 0.5 hr | 0.75 hr | 1.0 hr | 2.0 hr |
| 30% drug loading | 0.84 | 0.86 | 0.46 | 0.44 | 0.25 |
| 40% drug loading | 0.83 | 0.85 | 0.47 | 0.46 | 0.43 |
| 50% drug loading | 0.83 | 0.85 | 0.45 | 0.41 | 0.25 |

### Example 7-4 Four-week solid stability study of solid dispersions with the formulation of API+HPC SSL

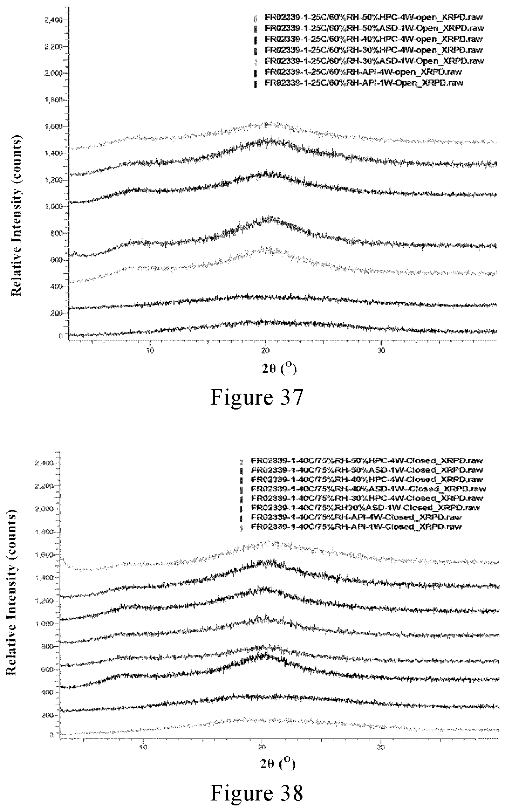

All solid dispersions with formulation of API+HPC SSL were stored at 25 °C/60%RH (open) and 40 °C/75%RH (sealed). After one week and four weeks (1w and 4w), the sample appearance was visually observed, the crystal form change was tested by XRPD, and the sample purity was analyzed by HPLC. The results are shown in Tables 11 to 12 and Figures 37 to 38. The analysis results showed that three solid dispersions all showed great solid stability.

**Table 11 HPLC results of four-week solid stability of API+HPC SSL solid dispersions with different drug loading**

| Samples | Conditions | Time | Impurities % | Contents % | RRT (%) | | | | | | |
|---|---|---|---|---|---|---|---|---|---|---|---|
| | | | | | 0.92 | 0.93 | 0.95 | 0.96 | 1.00 | 1.10 | 1.17 |
| API control | Initial | | 0.78 | / | 0.05 | 0.10 | 0.09 | 0.15 | 99.22 | 0.31 | 0.09 |
| | 25 °C/60%RH open | 1w | 0.81 | 96.84 | 0.05 | 0.10 | 0.09 | 0.14 | 99.20 | 0.32 | 0.10 |
| | | 4w | 0.70 | 101.1 | <LOQ | 0.07 | 0.09 | 0.14 | 99.30 | 0.32 | 0.09 |
| | 40 °C/75%RH sealed | 1w | 0.75 | 97.46 | 0.03 | 0.06 | 0.09 | 0.15 | 99.25 | 0.32 | 0.09 |
| | | 4w | 0.70 | 101.2 | <LOQ | 0.06 | 0.09 | 0.14 | 99.30 | 0.33 | 0.09 |
| 30% drug loading | Initial | | 0.74 | 103.3 | 0.05 | 0.09 | 0.12 | 0.10 | 99.26 | 0.30 | 0.08 |
| | 25 °C/60%RH open | 1w | 0.82 | 100.2 | 0.05 | 0.12 | 0.11 | 0.12 | 99.18 | 0.32 | 0.10 |
| | | 4w | 0.73 | 105.0 | <LOQ | 0.06 | 0.12 | 0.11 | 99.27 | 0.36 | 0.08 |
| | 40 °C/75%RH sealed | 1w | 0.70 | 101.9 | 0.02 | 0.04 | 0.11 | 0.12 | 99.30 | 0.32 | 0.09 |
| | | 4w | 0.73 | 105.5 | <LOQ | 0.11 | 0.11 | 0.11 | 99.27 | 0.31 | 0.08 |
| 40% drug loading | Initial | | 0.76 | 100.9 | 0.05 | 0.10 | 0.12 | 0.11 | 99.24 | 0.30 | 0.09 |
| | 25 °C/60%RH open | 1w | 0.80 | 98.54 | 0.05 | 0.10 | 0.11 | 0.12 | 99.20 | 0.32 | 0.10 |
| | | 4w | 0.71 | 103.7 | <LOQ | 0.04 | 0.11 | 0.12 | 99.29 | 0.35 | 0.09 |
| | 40 °C/75%RH sealed | 1w | 0.69 | 99.76 | 0.01 | 0.04 | 0.10 | 0.13 | 99.31 | 0.32 | 0.09 |
| | | 4w | 0.73 | 103.32 | <LOQ | 0.11 | 0.11 | 0.11 | 99.27 | 0.31 | 0.09 |
| 50% drug loading | Initial | | 0.77 | 100.6 | 0.06 | 0.12 | 0.12 | 0.10 | 99.23 | 0.29 | 0.09 |
| | 25 °C/60%RH open | 1w | 0.78 | 98.29 | 0.05 | 0.10 | 0.10 | 0.12 | 99.22 | 0.31 | 0.10 |
| | | 4w | 0.71 | 102.8 | <LOQ | 0.05 | 0.11 | 0.12 | 99.29 | 0.35 | 0.09 |
| | 40 °C/75%RH sealed | 1w | 0.67 | 99.07 | 0.01 | 0.03 | 0.10 | 0.13 | 99.33 | 0.31 | 0.09 |
| | | 4w | 0.81 | 102.2 | 0.05 | 0.15 | 0.11 | 0.11 | 99.19 | 0.31 | 0.09 |

**Table 12 Appearance characteristics of solid dispersions with different drug loadings under two stability test conditions for one week and four weeks**

| Samples | Appearance | | | | |
|---|---|---|---|---|---|
| | Initial | 25 °C/60%RH (open) | | 40 °C/75%RH (sealed) | |
| | | One week | Four weeks | **One** week | Four weeks |
| API control | White powder | White powder | Slight agglomeration | White powder | Slight agglomeration |
| 30% drug loading | White powder | White powder | Slight agglomeration | White powder | Slight agglomeration |
| 40% drug loading | White powder | White powder | Slight agglomeration | White powder | Slight agglomeration |
| 50% drug loading | White powder | White powder | Slight agglomeration | White powder | Slight agglomeration |

### Example 8 Preparation of solid dispersion using the crystalline form G of the compound of formula A as raw material

### Example 8-1 Characteristics of crystalline form G of Compound A

The solid dispersion of the present invention may be prepared using amorphous or other crystalline forms as raw materials, in addition to the API control mentioned above. For example, crystal form G of the compound of formula A may be used as a raw material.

Preparation method. The compound of formula A was prepared by referring to the method of Example 15 of PCT/CN2022/097236, and then the compound was ring-closed under acidic conditions and crystallized in acetonitrile-water to obtain the crystalline form G of the compound of formula A.

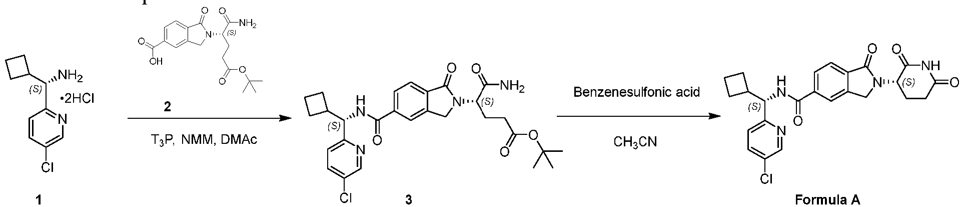

Compound 1 (32.5 g, 121 mmol) and compound 2 (56 g, 155 mmol) were added to N,N-dimethylacetamide (DMAc) (500 mL), and propylphosphoric anhydride (T₃P) (46.7 g, 147 mmol) and N-methylmorpholine (NMM) (62 g, 613 mmol) were added. The mixture was degassed and purged with N₂ for 3 times and stirred at 25 °C under N₂ atmosphere for 12 hours. The mixture was poured into a saturated sodium chloride aqueous solution (1000 mL), then filtered and washed with water (1000 mL). The filter cake was dissolved in DCM (2000 mL), washed with saturated aqueous NaHCO₃ solution (1000 mL). The organic phase was dried with anhydrous Na₂SO₄, filtered and concentrated under reduced pressure to obtain compound 3 (66 g) as a white solid (HPLC purity greater than 98%, chiral purity greater than 98%).

Compound 3 (66 g) was dissolved in CH₃CN (660 mL) and benzenesulfonic acid (57.4 g, 363 mmol) was added. The mixture was stirred at 50 °C for 16 hours under N₂ atmosphere, cooled to 0 °C, and the pH was adjusted to 7-8 with 7% NaHCO₃. The above solution was slowly added dropwise to water (3300 mL), and the mixture was stirred for 3 h, suction filtered, and the filter cake was washed with water (300 mL). The filter cake was vacuum dried to obtain compound A (50 g) as a white solid.

The yield of the two steps was 89%.

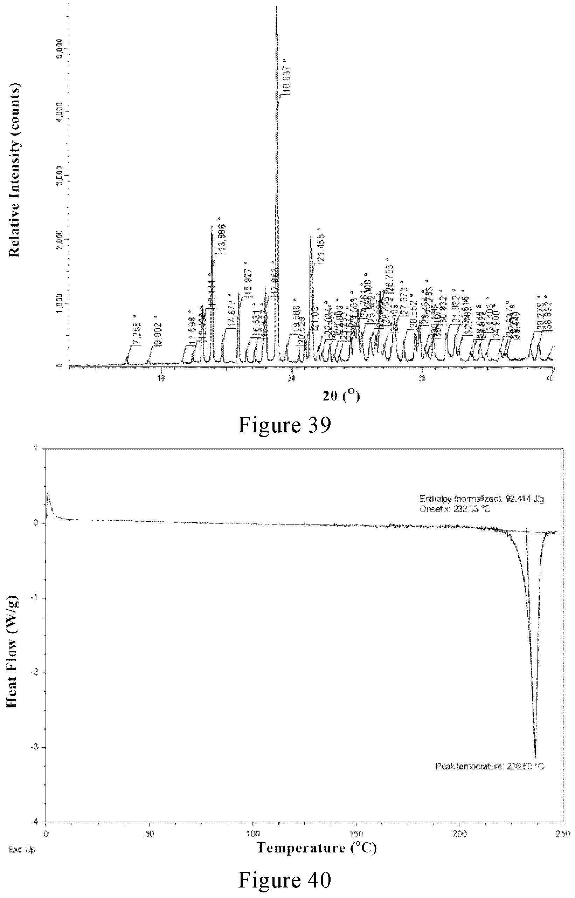

The compound of formula A above was identified as crystalline form G by XRPD (Figure 39).

It has XRPD characteristic peaks at positions substantially as shown in the following Table 13 and/or an X-ray powder diffraction (XRPD) pattern substantially as shown in Figure 39. It has at least three, at least four, at least five or at least six characteristic peaks at the following positions in an X-ray powder diffraction (XRPD) pattern in 2θ angles: 18.837 ± 0.2°, 13.886 ± 0.2°, 21.455 ± 0.2°, 26.755 ± 0.2°, 15.927 ± 0.2° and 15.953 ± 0.2°. The parameters of each peak are shown in Table 13.

**Table 13 XRPD characteristic peaks of crystalline form G of the compound of formula A**

| Index | Angle | Intensity | Rel. Intensity |
|---|---|---|---|
| 1 | 7.355 ° | 94.2941 | 1.6% |
| 2 | 9.002 ° | 83.7517 | 1.1% |
| 3 | 11.598 ° | 86.9896 | 0.7% |
| 4 | 12.430° | 159.645 | 2.3% |
| 5 | 13.141 ° | 676.318 | 15.2% |
| 6 | 13.886 ° | 1545.5 | 37.2% |
| 7 | 14.673 ° | 356.66 | 7.2% |
| 8 | 15.927 ° | 859.852 | 20.1% |
| 9 | 16.531 ° | 211.2 | 3.8% |
| 10 | 17.137 ° | 208.944 | 3.6% |
| 11 | 17.953° | 869.03 | 19.9% |
| 12 | 18.837° | 4034.74 | 100.0% |
| 13 | 19.586 ° | 276.525 | 5.0% |
| 14 | 20.529 ° | 97.8431 | 0.6% |
| 15 | 21.031 ° | 331.751 | 6.3% |
| 16 | 21.455 ° | 1391.08 | 33.0% |
| 17 | 22.034 ° | 241.296 | 3.9% |
| 18 | 22.312 ° | 184.232 | 2.5% |
| 19 | 22.896 ° | 270.145 | 4.7% |
| 20 | 23.233 ° | 163.817 | 2.0% |
| 21 | 23.647 ° | 157.872 | 1.7% |
| 22 | 24.503 ° | 393.162 | 7.4% |
| 23 | 24.761 ° | 519.971 | 10.5% |
| 24 | 25.068 ° | 677.033 | 14.4% |
| 25 | 25.362 ° | 434.118 | 8.2% |
| 26 | 25.997 ° | 361.374 | 6.2% |
| 27 | 26.455 ° | 411.917 | 7.4% |
| 28 | 26.755 ° | 918.346 | 20.2% |
| 29 | 27.109° | 293.309 | 4.4% |
| 30 | 27.873 ° | 558.659 | 11.3% |
| 31 | 28.552 ° | 343.613 | 6.1% |
| 32 | 29.454 ° | 413.626 | 7.7% |
| 33 | 29.783 ° | 571.263 | 11.6% |
| 34 | 30.186° | 267.033 | 3.9% |
| 35 | 30.407 ° | 203.296 | **2.3%** |
| 36 | 30.832° | 419.995 | 7.9% |
| 37 | 31.832 ° | 428.625 | 8.2% |
| 38 | 32.516 ° | 416.376 | 8.0% |
| 39 | 32.783 ° | 252.051 | 3.9% |
| 40 | 33.646 ° | 186.674 | 2.3% |
| 41 | 33.822 ° | 179.265 | 2.1% |
| 42 | 34.403 ° | 300.504 | 5.1% |
| 43 | 34.900 ° | 190.403 | 2.4% |
| 44 | 35.937 ° | 236.792 | 3.7% |
| 45 | 36.233 ° | 176.214 | 2.1% |
| 46 | 36.440 ° | 175.915 | 2.0% |
| 47 | 38.278 ° | 319.277 | 5.3% |
| 48 | 38.892 ° | 326.188 | 5.6% |
| 49 | 39.595 ° | 134.695 | 0.8% |

As shown in Figure 40, it has an endothermic peak at 236.59 ± 2.0 ° C in the DSC pattern.

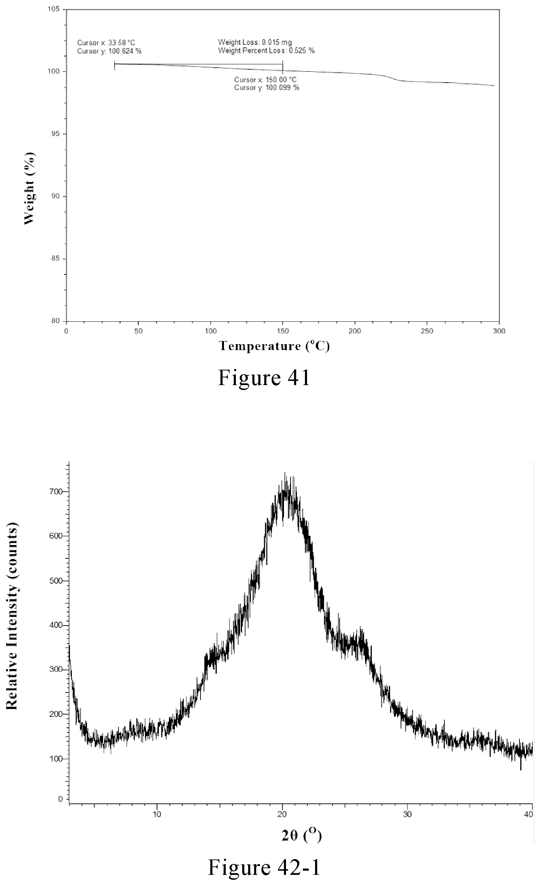

In the thermogravimetric analysis (TGA) of Figure 41, it has a weight loss of 0.52 wt% before reaching 150.00 ± 2.0 °C.

Example 8-2 Preparation of solid dispersion of crystalline form G of the compound of formula A

The spray-drying parameters were set according to Table 14, and two batches of appropriate amounts of crystalline form G of the compound of formula A, and the polymer carrier (HPC SSL, w/w) were weighed, and acetone: water = 9:1 (v/v) solvent was added. All samples were then stirred at 700 rpm until completely dissolved, and then spray dried by Buchi B-290 to obtain solid powder.

**Table 14 Spray drying parameters of solid dispersions**

| Parameter settings | Formulation: crystalline form G of the compound of formula A: HPC SSL | |
|---|---|---|
| Solvents | Acetone: H₂O=9:1 | |
| Target drug loading % (w/w) | 50% | |
| API concentration (mg/mL) | 10 | 7 |
| Inlet temperature (°C) | 100 | |
| Pump speed % | 40 | |
| Inhalation % | 100 | |
| Flow | 35 | |
| Actual inlet temperature (°C) | 95~99 | 99~103 |
| Outlet (°C) | 65~67 | 63~66 |

The solid powder in Table 14 was further dried at 60 °C under vacuum conditions for 24 hours to obtain a solid dispersion sample, which was then characterized and tested by XRPD, TGA, mDSC, PSD, GC, chiral purity and HPLC.

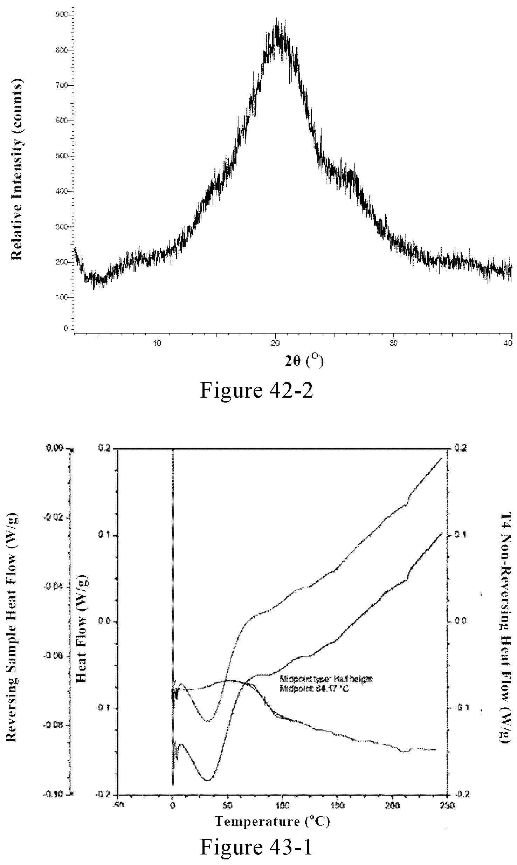

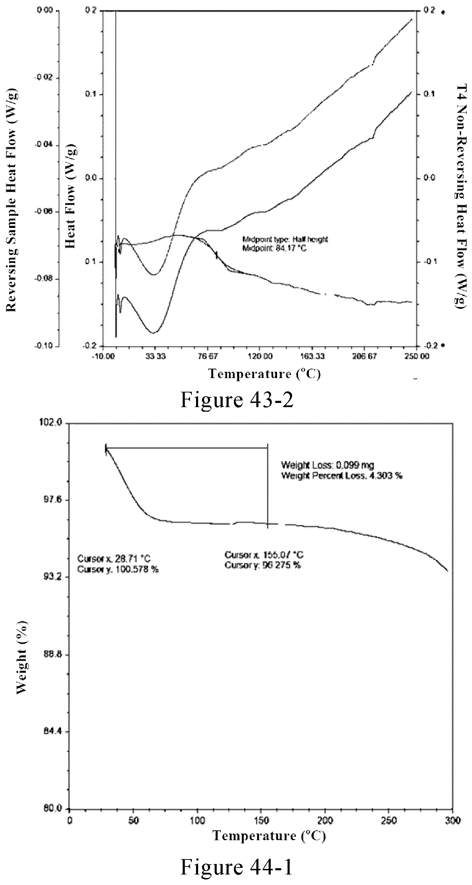

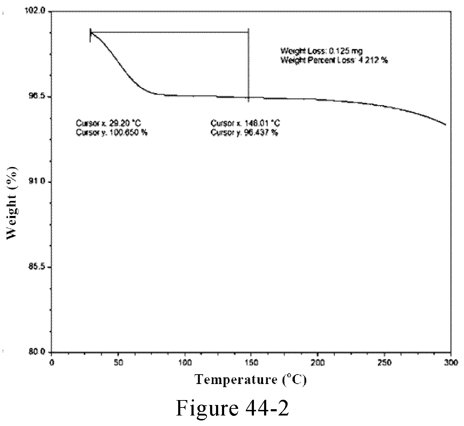

The results are shown in Table 15, Figures 42-1 and 42-2. Two batches of XRPD showed that all solid dispersions are amorphous, as shown in Figures 43-1 and 43-2. mDSC showed that Tg (glass transition temperature) was 82~85 °C. As shown in Figures 44-1 and 44-2, the solid dispersion has a weight loss of 4.21~4.30 wt% at 148~155 °C. The content of the solid dispersion was 99.34%~99.77%; there was no significant change in purity, and the HPLC purity was about 99.35%~99.37%; there was no significant increase in isomers. The particle size D₉₀ of the solid dispersion was 18.00~20.00 µm.

**Table 15 Characterization results of solid dispersions prepared by spray-drying**

| Formulations | | Solid dispersion dry powder with 50% drug loading | Solid dispersion dry powder with 50% drug loading |
|---|---|---|---|
| XRPD | | Amorphous | Amorphous |
| TGA | | 4.30 | 4.21 |
| mDSC | | 84.17 | 82.93 |
| HPLC | Drug loading (%) | 49.89 | 49.17 |
| | Purity (%) | 99.35 | 99.37 |
| | Content (%) | 99.77 | 98.34 |
| PSD | D₁₀ (µm) | 2.85 | 2.99 |
| | D₅₀ (µm) | 7.00 | 7.37 |
| | D₉₀ (µm) | 18.70 | 19.40 |
| Gas chromatog | Acetone | 125.92 | 10.1 |
| raphy of solvent residue (ppm) | | | |
| Chiral purity (%) | Purity (%) | 97.49 | 97.66 |
| | Chiral impurities (%) | 2.51 | 2.34 |

### Example 9 In vivo pharmacokinetic study of solid dispersions

According to Table 16, three solid dispersions, namely, formulation of API+Soluplus, formulation of API+PVP VA64 and formulation of API+HPC SSL, were selected to conduct *in vivo* pharmacokinetic tests with the corresponding amorphous form of API to study the pharmacokinetic characteristics of solid dispersions with different formulations in animals.

Method: solid dispersions with different formulations were evaluated with the pharmacokinetic characteristics of male Sprague Dawley (SD) rats after intragastric administration.

Materials: SD rats (male, 200-250 g, 6-8 weeks old, Zhejiang Weitong Lihua).

Steps: the solid dispersion with the formulation of Table 16 was tested by intragastric administration of 300 mg/kg (dosage calculated according to the compound of formula A) to SD rats, and the solvent was 25 mM citrate buffer pH3 containing 5% Tween 80. The animals were free to eat and drink water. Blood was collected from the jugular vein 15 min (0.25 h), 30 min (0.5 h), 60 min (1 h), 2 h, 4 h, 6 h, 8 h and 24 h after administration. Whole blood was collected in an anticoagulant tube containing EDTA-K2, and after thorough mixing, it was centrifuged at 4 °C and 4000 g for 5 minutes to separate plasma. Plasma samples were stored at -75 ± 15 °C until the blood concentration of the compound of formula A was determined by LC-MS/MS. The relevant pharmacokinetic parameters were calculated using the non-compartmental model and linear/log trapezoidal method of Phoenix WinNonlin 6.1.

As shown in Table 16 and Figure 22, 20% API+HPC SSL (i.e., API+HPC SSL (1:4, w/w)) has the highest plasma concentration (Cₘₐₓ =101,467 ng/mL) at 1.67 hours (Tₘₐₓ), and AUCₗₐₛₜ was 647,047 h*ng/mL, which was significantly higher than other solid dispersion samples.

**Table 16 PK results of solid dispersions with different formulations containing 20% drug loading**

| PK parameters | HPC SSL_20% | PVP VA64_20% | Soluplus_20% | Amorphous form of API |
|---|---|---|---|---|
| Dosing volume (mL/kg) | 20 | 20 | 20 | 10 |
| T_{1/2} (h) | 3.30 | 6.0 | 5.48 | 4.06 |
| Tₘₐₓ (h) | 1.67 | 0.83 | 0.83 | 1.00 |
| Cₘₐₓ (ng/mL) | 101,467 | 88,733 | 60,267 | 18,700 |
| AUCₗₐₛₜ(h*ng/mL) | 647,047 | 318,700 | 203,402 | 110,592 |
| AUC_{Inf}(h*ng/mL) | 652,391 | 327,132 | 207,309 | 112,251 |
| AUCₗₐₛₜ/D (h*mg/mL) | 2,157 | 1,062 | 678 | 369 |
| Fold increase in AUC (compared to amorphous form of API) | 5.85 | 2.88 | 1.84 | / |

To further confirm the PK effect of optimized drug loading, API+HPC SSL solid dispersion was used as an example to conduct PK experiments on API+HPC SSL solid dispersions with different drug loading. The results are shown in Table 17 and Figure 23, 50% ASD-HPC SSL (i.e., API+HPC SSL (1:1, w/w)) had the highest plasma concentration (Cₘₐₓ =115,000 ng/mL) at 1.00 hour (Tₘₐₓ), and AUCₗₐₛₜ was 508,555 h*ng/mL, which was significantly higher than solid dispersion samples with other drug loading.

**Table 17 PK results of API+HPC SSL solid dispersions with different drug loading**

| PK parameters | HPC SSL 20% | HPC SSL_30% | HPC SSL_40% | HPC SSL_50% |
|---|---|---|---|---|
| Dosing volume (mL/kg) | 20 | 10 | 10 | 10 |
| T_{1/2} (h) | 3.30 | 5.00 | 3.13 | 6.12 |
| Tₘₐₓ (h) | 1.67 | 2.00 | 2.00 | 1.00 |
| Cₘₐₓ (ng/mL) | 101,467 | 62,933 | 77,200 | 115,000 |
| AUCₗₐₛₜ (h*ng/mL) | 647,047 | 410,574 | 385,211 | 508,555 |
| AUC_{Inf} (h*ng/mL) | 652,391 | 489,313 | 386,420 | 524,442 |
| AUₗₐₛₜ/D (h*mg/mL) | 2,157 | 1,369 | 1,284 | 1,695 |
| Fold increase in AUC (compared to amorphous form of API) | 5.85 | 3.71 | 3.48 | 4.60 |

### Conclusion:

1. The solid dispersion comprising the active ingredient and a carrier prepared by the present invention has great solid stability.
2. The solid dispersion comprising the active ingredient prepared by the present invention can significantly improve the oral bioavailability of the active ingredient. In particular, when the solid dispersion of the present invention is administrated, the area under the curve of the active ingredient in plasma is 1.5 to 7 times, preferably 5 to 6 times, the area under the curve when the active ingredient is administered alone.

All literatures mentioned in the present invention are incorporated by reference herein, as though individually incorporated by reference. Additionally, it should be understood that after reading the above teaching, many variations and modifications may be made by the skilled in the art, and these equivalent forms also fall within the scope defined by the appended claims.

## Claims

1. A solid dispersion, wherein the solid dispersion comprises an active ingredient and a carrier, wherein:
the active ingredient is one or more of a compound of formula A or a derivative, a crystalline form, an amorphous form or a pharmaceutically acceptable salt, a hydrate or a solvate thereof, 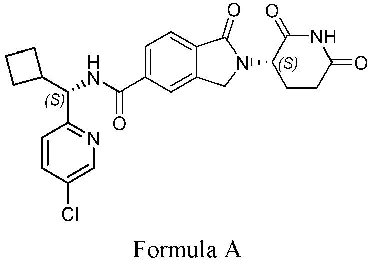
the carrier is selected from one or more of homopolymer and copolymer of N-vinyl lactam, cellulose derivative, graft copolymer, polyalkylene oxide with a high molecular weight, polyacrylate and polymethacrylate, polyacrylamide, polyvinyl acetate, oligosaccharide or polysaccharide or copolymer thereof, and acrylic acid copolymer.

2. The solid dispersion according to claim 1, wherein the homopolymer and copolymer of N-vinyl lactam is povidone (PVP), or a copolymer of PVP and polyvinyl acetate; preferably, the povidone is PVP K30; preferably, the copolymer of PVP and polyvinyl acetate is PVP VA64;
and/or, the cellulose derivative is hydroxypropyl methylcellulose acetate succinate, hydroxypropyl methylcellulose, or hydroxypropyl cellulose, preferably, the hydroxypropyl methylcellulose acetate succinate is one or more of HPMCAS 716G, HPMCAS 912G, HPMCAS 126G, HPMCAS 716F, HPMCAS 912F, HPMCAS 126F, HPMCAS LG, HPMCAS MG, HPMCAS HG, HPMCAS LF, HPMCAS MF and HPMCAS HF; preferably, the hydroxypropyl methylcellulose is HPMC E3; preferably, the hydroxypropyl cellulose is HPC SSL;
and/or, the acrylic copolymer is a methacrylic acid copolymer and methacrylate copolymer; preferably, the methacrylic acid copolymer and the methacrylate copolymer is Eudragit; preferably, the Eudragit is one or more selected from Eudragit L100, Eudragit E100, Eudragit S100, Eudragit L100-55, and Eudragit EPO;
and/or, the graft copolymer is Soluplus;
and/or, the pharmaceutically acceptable salt of the compound of formula A is hydrochloride, hydrobromide, hydroiodide, sulfate, bisulfate, 2-hydroxyethanesulfonate, phosphate, hydrogenphosphate, acetate, adipate, alginate, lysine, arginine, histidine, aspartate, benzoate, butyrate, camphorate, camphorsulfonate, digluconate, glycerophosphate, hemisulfate, heptanoate, hexanoate, formate, succinate, fumarate, maleate, ascorbate, isethionate, salicylate, methanesulfonate, mesitylenesulfonate, naphthalenesulfonate, nicotinate, 2-naphthalenesulfonate, oxalate, pamoate, pectinate, persulfate, 3-phenylpropionate, picrate, pivalate, propionate, trichloroacetate, trifluoroacetate, glutamate, bicarbonate, undecanoate, lactate, citrate, tartrate, gluconate, edisylate, benzenesulfonate, L-tartrate, maleate, sodium salt, potassium salt, choline salt, tromethamine salt, calcium salt or p-toluenesulfonate, preferably phosphate, sulfate, L-tartrate, hydrochloride, maleate, hydrobromide, methanesulfonate, lysine salt, arginine salt, histidine salt, sodium salt, potassium salt, choline salt, tromethamine salt, or calcium salt;
and/or, the hydrate of the compound of formula A is hemihydrate, monohydrate, dihydrate, trihydrate, tetrahydrate, pentahydrate, hexahydrate, heptahydrate, octahydrate, nonahydrate, decahydrate, undecahydrate or dodecahydrate.

3. The solid dispersion according to any one of claims 1 to 2, wherein the carrier is selected from one or more of polyvidone, copolymer of PVP and polyvinyl acetate, hydroxypropyl methylcellulose acetate succinate, hydroxypropyl methylcellulose, hydroxypropyl cellulose, acrylic acid copolymer and graft copolymer;
preferably, the carrier is selected from one or more of PVP K30, PVP VA64, Soluplus, HPMC E3, HPMCAS MG, HPMCAS HG, HPC SSL, and Eudragit L100;
preferably, the carrier is selected from one or more of PVP VA64, Soluplus and HPC SSL;
preferably, the carrier is HPC SSL.

4. The solid dispersion according to any one of claims 1 to 3, wherein the weight ratio of the active ingredient to the carrier is 0.1:10 ~ 10:0.1.

5. The solid dispersion according to any one of claims 1 to 4, wherein the weight ratio of the active ingredient to the carrier is 1:4, 3:7, 4:6 or 1: 1.

6. The solid dispersion according to any one of claims 1 to 5, wherein the carrier is HPC SSL, and the weight ratio of the active ingredient to HPC SSL is 1:4, 3:7, 4:6 or 1: 1.

7. The solid dispersion according to any one of claims 1 to 4, wherein the solid dispersion is solid dispersion 1, which comprises the compound of formula A and Soluplus in a weight ratio of 1:4, and the solid dispersion 1 is amorphous and has an XRPD pattern substantially as shown in Figure 8;
or the solid dispersion is solid dispersion 2, which comprises the compound of formula A and PVP VA64 in a weight ratio of 1:4, and the solid dispersion 2 is amorphous and has an XRPD pattern substantially as shown in Figure 12;
or the solid dispersion is solid dispersion 3, which comprises the compound of formula A and HPC SSL in a weight ratio of 1:4, and the solid dispersion 3 is amorphous and has an XRPD pattern substantially as shown in Figure 16;
preferably, the solid dispersion 1 further optionally has one or more of the following features:
1) having a weight loss of 0.95 wt% before the temperature reaches 180.00 ± 2.0 °C in TGA pattern;
2) having a glass transition temperature at 99.79 ± 2.0 °C in mDSC pattern;
3) having a TGA pattern substantially as shown in Figure 9; and/or
4) having a mDSC pattern substantially as shown in Figure 10;
preferably, the solid dispersion 2 further optionally has one or more of the following features:
1) having a weight loss of 1.89 wt% before the temperature reaches 210 ± 2.0 °C in TGA pattern;
2) having a glass transition temperature at 118.74 ± 2.0 °C in mDSC pattern;
3) having a TGA pattern substantially as shown in Figure 13; and/or
4) having a mDSC pattern substantially as shown in Figure 14;
preferably, the solid dispersion 3 further optionally has one or more of the following features:
1) having a weight loss of 0.99 wt% before the temperature reaches 180 ± 2.0 °C in TGA pattern;
2) having a glass transition temperature at 70.72 ± 2.0 °C in mDSC pattern;
3) having a TGA pattern substantially as shown in Figure 17; and/or
4) having a mDSC pattern substantially as shown in Figure 18.

8. The solid dispersion according to any one of claims 1 to 7, wherein the area under the curve (AUC) of the active ingredient in the solid dispersion is 1.5 to 7 times, preferably 5 to 6 times, the AUC of the active ingredient when administered alone.

9. A method for preparing the solid dispersion according to any one of claims 1 to 8, wherein the method comprises the following steps:
method 1
mixing one or more of the compound of formula A or a derivative, a crystalline form, an amorphous form thereof, or a pharmaceutically acceptable salt, a hydrate, or a solvate thereof, optionally with other components, with a solvent to form a solution or suspension, and removing the solvent to obtain the solid dispersion; or
method 2
mixing one or more of the compound of formula A or a derivative, a crystalline form, an amorphous form thereof, or a pharmaceutically acceptable salt, a hydrate, or a solvate thereof, optionally with other components, heating and then extruding to obtain the solid dispersion; or
method 3
mixing one or more of the compound of formula A or a derivative, a crystalline form, an amorphous form thereof, or a pharmaceutically acceptable salt, a hydrate, or a solvate thereof, optionally with other components, with a solvent and spray-drying to obtain the solid dispersion.

10. The method according to claim 9, wherein in the method 1 or the method 3,
the solvent is one or more selected from water, alcohol solvent, ester solvent, ketone solvent, halogenated hydrocarbon solvent, nitrile solvent and ether solvent, wherein the alcohol solvent is preferably ethanol and/or methanol; the ester solvent is preferably ethyl acetate; the ketone solvent is preferably acetone; the halogenated hydrocarbon solvent is preferably dichloromethane; the nitrile solvent is preferably acetonitrile; the ether solvent is preferably tetrahydrofuran; preferably, the solvent is dichloromethane and/or ethanol; preferably, the solvent is acetone and/or water;
and/or, the weight to volume ratio of the "one or more selected from the compound of formula A or a derivative, a crystalline form, an amorphous form thereof, or a pharmaceutically acceptable salt, a hydrate or a solvate thereof" to the solvent is (0.1~30):1 mg/mL, preferably (1~10):1 mg/mL, and more preferably 5:1.5 mg/mL;
and/or, the solvent is an alcohol solvent and/or a halogenated hydrocarbon solvent, wherein the alcohol solvent is preferably methanol or ethanol; the halogenated hydrocarbon solvent is preferably dichloromethane; preferably, the solvent is dichloromethane and methanol, or, dichloromethane and ethanol, wherein the volume ratio of dichloromethane to methanol, or, dichloromethane to ethanol is preferably 9:1~1:1;
and/or, the solvent is acetone and water, wherein the volume ratio of acetone to water is preferably 7:1~10:1;
and/or, the inlet temperature of spray-drying air is set to 40 °C ~ 200 °C, preferably 80 °C ~ 120 °C.

11. A pharmaceutical composition, wherein the pharmaceutical composition comprises:
(1) the solid dispersion according to any one of claims 1 to 8; and
(2) a pharmaceutically acceptable excipient.

12. A pharmaceutical formulation comprising the pharmaceutical composition according to claim 11; wherein the pharmaceutical formulation may be a solid formulation, and the dosage form of the solid formulation is selected from a group consisting of: powder, granule, tablet, capsule, pill and film.

13. Use of the solid dispersion according to any one of claims 1 to 8 in the manufacture of a medicament for treating a proliferative disease.

14. A method for treating a proliferative disease, wherein the method comprises administering to a subject in need thereof a therapeutically effective amount of the solid dispersion according to any one of claims 1 to 8.

15. The use according to claim 13 or the method according to claim 14, wherein the proliferative disease is selected from: breast cancer, colon cancer, brain cancer, prostate cancer, kidney cancer, pancreatic cancer, ovarian cancer, head and neck cancer, melanoma, colorectal cancer, gastric cancer, squamous cell carcinoma, small cell lung cancer, non-small cell lung cancer, testicular cancer, Merkel cell carcinoma, glioblastoma, neuroblastoma, lymphoid organ cancer and hematological malignancy including leukemia (acute lymphoblastic leukemia (ALL), acute myeloid leukemia (AML), chronic lymphocytic leukemia (CLL), chronic myeloid leukemia (CML), acute monocytic leukemia (AMOL), hairy cell leukemia (HCL), T cell prolymphocytic leukemia (T-PLL), large granular lymphocytic leukemia, adult T cell leukemia), lymphoma (small lymphocytic lymphoma (SLL), Hodgkin's lymphoma (nodular sclerosis, mixed cellular, lymphocyte-rich, lymphocyte depleted or not depleted, and nodular lymphocyte-predominant Hodgkin lymphoma), non-Hodgkin's lymphoma (all subtypes), chronic lymphocytic leukemia/small lymphocytic lymphoma, B cell prolymphocytic leukemia, lymphoplasmacytic lymphoma (such as Waldenström macroglobulinemia), splenic marginal zone lymphoma, plasma cell neoplasms (plasma cell myeloma, plasmacytoma, monoclonal immunoglobulin deposition disease, heavy chain disease), extranodal marginal zone B cell lymphoma (MALT lymphoma), lymphoma (NMZL), follicular lymphoma, mantle cell lymphoma, diffuse large B cell lymphoma, mediastinal (thymic) large B cell lymphoma, intravascular large B cell lymphoma, primary effusion lymphoma, Burkitt lymphoma/leukemia, T cell prolymphocytic leukemia, T cell large granular lymphocytic leukemia, aggressive NK cell leukemia, adult T cell leukemia/lymphoma, extranodal NK/T cell lymphoma (nasal type), enteropathy-type T cell lymphoma, hepatosplenic T cell lymphoma, blastic NK cell lymphoma, mycosis fungoides/Sezary syndrome, primary cutaneous CD30-positive T cell lymphoma disease, primary cutaneous anaplastic large cell lymphoma, lymphomatoid papulosis, angioimmunoblastic T cell lymphoma, peripheral T cell lymphoma (unspecified), anaplastic large cell lymphoma, or multiple myeloma (plasma cell myeloma or Kahler's disease).
